# EUROPÄISCHE PATENTSCHRIFT

(11) **EP 1 742 931 B1**
(45) Veröffentlichungstag und Bekanntmachung des Hinweises auf die Patenterteilung: **23.04.2014**
(21) Anmeldenummer: 05716210.9
(22) Anmeldetag: 18.03.2005
(51) Int. Cl.: C07D 307/91

(54) **DIBENZOFURAN-, DIBENZOTHIOPHEN- UND FLUORENDERIVATE ALS KOMPONENTEN IN FLÜSSIGKRISTALLMISCHUNGEN**
DIBENZOFURAN, DIBENZOTHIOPHENE AND FLUORENE DERIVATIVES AS COMPONENTS OF LIQUID-CRYSTALLINE MIXTURES
DERIVES DE DIBENZOFURANE, DE DIBENZOTHIOPHENE ET DE FLUORENE COMME COMPOSANTS DE MELANGES LIQUIDES CRISTALLINS

(30) Priorität: 14.04.2004 DE 102004018526
(43) Veröffentlichungstag der Anmeldung: 17.01.2007
(73) Patentinhaber: Merck Patent GmbH, 64293 Darmstadt (DE)
(72) Erfinder: LIETZAU, Lars, 64295 Darmstadt (DE); BREMER, Matthias, 64295 Darmstadt (DE); KLASEN-MEMMER, Melanie, 67259 Heuchelheim (DE)
(86) Internationale Anmeldenummer: PCT/EP2005/002922
(87) Internationale Veröffentlichungsnummer: WO 2005/105772

(56) Entgegenhaltungen:
- EP-A- 1 201 632
- EP-A- 1 223 209
- WO-A-02/055463
- DE-A1- 2 114 461
- JP-A- 2001 064 216
- US-A- 2 146 730
- US-A- 3 953 602

## Beschreibung

Die vorliegende Erfindung betrifft Dibenzofuran-, Dibenzothiophen- und Fluorenderivate, flüssigkristalline Medien enthaltend diese Derivate sowie elektrooptische Anzeigeelemente enthaltend diese flüssigkristallinen Medien. Insbesondere betrifft die Erfindung Dibenzofuran-, Dibenzothiophen- und Fluorenderivate mit negativer dielektrischer Anisotropie.

Flüssigkristalle haben ein breites Anwendungsfeld gefunden, seitdem vor etwa 30 Jahren die ersten kommerziell anwendbaren flüssigkristallinen Verbindungen gefunden wurden. Bekannte Anwendungsgebiete sind insbesondere Anzeigedisplays für Uhren und Taschenrechner sowie große Anzeigetafeln, wie sie in Bahnhöfen, Flughäfen und Sportarenen verwendet werden. Weitere Anwendungsgebiete sind Displays von tragbaren Computern und Navigationssystemen sowie Videoapplikationen. Insbesondere für die zuletzt genannten Anwendungen werden hohe Anforderungen an Schaltzeiten und den Kontrast der Abbildungen gestellt.

Die räumliche Ordnung der Moleküle in einem Flüssigkristall bewirkt, dass viele seiner Eigenschaften richtungsabhängig sind. Von Bedeutung für den Einsatz in Flüssigkristallanzeigen sind dabei insbesondere die Anisotropien im optischen, dielektrischen und elasto-mechanischen Verhalten. Je nachdem, ob die Moleküle mit ihren Längsachsen senkrecht oder parallel zu den beiden Platten eines Kondensators orientiert sind, hat dieser eine andere Kapazität; die Dielektrizitätskonstante ε des flüssigkristallinen Mediums ist also für die beiden Orientierungen verschieden groß. Substanzen, deren Dielektrizitätskonstante bei senkrechter Orientierung der Moleküllängsachsen zu den Kondensatorplatten größer ist als bei paralleler Anordnung, werden als dielektrisch positiv bezeichnet. Mit anderen Worten: Ist die Dielektrizitätskonstante ε_{∥} parallel zu den Moleküllängsachsen größer als die Dielektrizitätskonstante ε_{⊥} senkrecht zu den Moleküllängsachsen, so ist die dielektrische Anisotropie Δε = ε_{∥} - ε_{⊥} größer null. Die meisten Flüssigkristalle, die in herkömmlichen Displays Verwendung finden, fallen in diese Gruppe.

Für die dielektrische Anisotropie spielen sowohl die Polarisierbarkeit des Moleküls als auch permanente Dipolmomente eine Rolle. Beim Anlegen einer Spannung an das Display richtet sich die Längsachse der Moleküle so aus, dass die größere der dielektrischen Konstanten wirksam wird. Die Stärke der Wechselwirkung mit dem elektrischen Feld hängt dabei von der Differenz der beiden Konstanten ab. Bei kleinen Differenzen sind höhere Schaltspannungen erforderlich als bei großen. Durch den Einbau geeigneter polarer Gruppen, wie z.B. von Nitrilgruppen oder Fluor, in die Flüssigkristallmoleküle läßt sich ein weiter Bereich von Arbeitsspannungen realisieren.

Bei den in herkömmlichen Flüssigkristallanzeigen verwendeten flüssigkristallinen Molekülen ist das entlang der Moleküllängsachse orientierte Dipolmoment größer als das senkrecht zur Moleküllängsachse orientierte Dipolmoment. Bei den am weitesten verbreiteten TN-Zellen (abgeleitet aus dem Englischen: "twisted nematic", verdrillt nematisch) ist eine nur etwa 5 bis 10 µm dicke flüssigkristalline Schicht zwischen zwei planparallelen Glasplatten angeordnet, auf die jeweils eine elektrisch leitende, transparente Schicht aus Zinnoxid oder Indium-Zinnoxid (ITO) als Elektrode aufgedampft ist. Zwischen diesen Filmen und der flüssigkristallinen Schicht befindet sich eine ebenfalls transparente Orientierungsschicht, die meist aus einem Kunststoff (z.B. Polyimiden) besteht. Sie dient dazu, durch Oberflächenkräfte die Längsachsen der benachbarten kristallinen Moleküle in eine Vorzugsrichtung zu bringen, so dass sie im spannungsfreien Zustand einheitlich mit der gleichen Orientierung flach oder mit demselben kleinen Anstellwinkel (englisch: "tilt angle") auf der Innenseite der Displayfläche aufliegen. Auf der Außenseite des Displays sind zwei Polarisationsfolien, die nur linear polarisiertes Licht ein- und austreten lassen, in einer bestimmten Anordnung aufgebracht.

Mit Flüssigkristallen, bei denen das größere Dipolmoment parallel zur Längsachse des Moleküls orientiert ist, sind bereits sehr leistungsfähige Displays entwickelt worden. Dabei kommen meist Mischungen von 5 bis 20 Komponenten zum Einsatz, um einen ausreichend breiten Temperaturbereich der Mesophase sowie kurze Schaltzeiten und niedrige Schwellenspannungen zu erreichen. Schwierigkeiten bereitet jedoch noch die starke Blickwinkelabhängigkeit bei Flüssigkristallanzeigen, wie sie beispielsweise für Laptops verwendet werden. Die beste Abbildungsqualität läßt sich erreichen, wenn die Fläche des Displays senkrecht zur Blickrichtung des Betrachters steht. Wird das Display relativ zur Betrachtungsrichtung gekippt, verschlechtert sich die Abbildungsqualität unter Umständen drastisch. Für einen höheren Komfort ist man bemüht, den Winkel, um den das Display von der Blickrichtung eines Betrachters ohne wesentliche Minderung der Abbildüngsqualität verkippt werden kann, möglichst groß zu gestalten. In jüngerer Zeit sind Versuche unternommen worden, zur Verbesserung der Blickwinkelabhängigkeit flüssigkristalline Verbindungen einzusetzen, deren Dipolmoment senkrecht zur Moleküllängsachse größer ist als parallel zur Längsachse des Moleküls. Die dielektrische Anisotropie Δε ist negativ. Im feldfreien Zustand werden diese Moleküle mit ihrer Längsachse senkrecht zur Glasfläche des Displays orientiert. Durch Anlegen eines elektrischen Feldes orientieren sie sich mehr oder weniger parallel zu den Glasflächen. Auf diese Weise konnte eine Verbesserung der Blickwinkelabhängigkeit erreicht werden. Derartige Displays werden als VA-TFT-Displays bezeichnet (abgeleitet aus dem Englischen: "vertically aligned").

Die Entwicklung auf dem Gebiet der flüssigkristallinen Materialien ist bei weitem noch nicht abgeschlossen. Zur Verbesserung der Eigenschaften flüssigkristalliner Anzeigeelemente ist man ständig bemüht, neue Verbindungen zu entwickeln, die eine Optimierung derartiger Displays ermöglichen.

In der WO 02/055463 A1 werden unter anderem 3-monosubstituierte und 3,7-disubstituierte 4,6-Difluordibenzofurane und -thiophene offenbart, ohne dass physikalische oder elektrooptische Eigenschaften präzisiert werden.

In der Druckschrift EP 1 201 632 A1 und EP 1 223 209 A1 werden fluorierte Fluorene als Teilstrukturen von Flüssigkristallkomponenten offenbart. Eine Fluorsubstitution gemäß der vorliegenden Erfindung wird nicht offenbart.

Die Druckschrift US 2,146,730 offenbart 1,8-Diioddibenzofuran, aber keine fluorierten Derivate.

Die Ofenlegungsschrift DE 2114461 offenbart die Verbindung 1,7-Dicyanofluoren.

Die Druckschriften JP 2001 064216 A und US 3,953,602 offenbaren Dibenzofuranverbindungen, die in den Positionen 1 und 8 keine Substituenten aufweisen.

Eine Aufgabe der vorliegenden Erfindung ist es daher, Verbindungen mit vorteilhaften Eigenschaften für den Einsatz in flüssigkristallinen Medien zur Verfügung zu stellen. Insbesondere sollten sie über eine negative dielektrische Anisotropie verfügen, was sie besonders geeignet macht für den Einsatz In flüssigkristallinen Medien für VA-Displays.

Diese Aufgabe wird erfindungsgemäß gelöst durch Verbindungen der allgemeinen Formel I 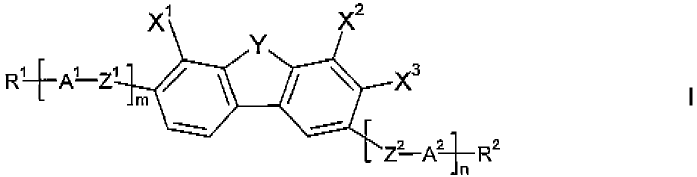 worin:
- m und n: jeweils unabhängig voneinander 0, 1, 2 oder 3 sind;
- Y: O, S, S(O), SO₂, CH₂, CF₂, CCl₂, CHF, CHCl, CFCl, C(CF₃)₂, CHCF₃, C(CN)₂ oder CHCN bedeutet;
- X¹, X² und X³: jeweils F bedeuten;
- A¹ und A²: jeweils unabhängig voneinander 1,4-Phenylen, worin =CH-ein- oder zweimal durch =N- ersetzt sein kann und das unsubstituiert oder ein- bis viermal unabhängig voneinander mit -CN, -F, -Cl, -Br, -I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substituiert sein kann, 1,4-Cyclohexylen, 1,4-Cyclohexenylen oder 1,4-Cyclo-hexadienylen, worin -CH₂- ein- oder zweimal unabhängig voneinander durch -O- oder -S- so ersetzt sein kann, dass Heteroatome nicht direkt verknüpft sind, und die unsubstituiert oder ein- oder mehrfach durch -F, -Cl, -Br und/oder -I substituiert sein können, bedeutet;
- Z¹ und Z²: jeweils unabhängig voneinander eine Einfachbindung, eine Doppelbindung, -CF₂O- -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- oder -C≡C-bedeuten;
- R¹ und R²: Wasserstoff, einen unsubstituierten, einen einfach mit -CN oder -CF₃ oder einen einfach oder mehrfach mit -F, -Cl, -Br und/oder -I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -COO-, -OCO- oder -O-CO-O- so ersetzt sein können, dass Heteroatome nicht direkt verknüpft sind, -F, -Cl, -Br, -I, -CN, -SCN, -NCS oder -SF₅ bedeuten;
wobei
- A¹, A², Z¹, Z²; R¹, R²: jeweils die gleiche oder unterschiedliche Bedeutungen haben können, wenn m beziehungsweise n größer als 1 sind.

Die Verbindungen besitzen ein negatives Δε und eignen sich daher insbesondere für eine Verwendung in VA-TFT-Displays. Vorzugsweise besitzen die erfindungsgemäßen Verbindungen ein Δε < -2 und besonders bevorzugt ein Δε < -4. Sie zeigen eine sehr gute Verträglichkeit mit den üblichen, in Flüssigkristallmischungen für Displays verwendeten Substanzen.

Ferner weisen die erfindungsgemäßen Verbindungen der Formel I insbesondere für die Verwendung in VA-TFT-Displays geeignete Werte der optischen Anisotropie Δn auf. Bevorzugt besitzen die erfindungsgemäßen Verbindungen ein Δn von größer als 0,05 und kleiner als 0,40. Auch die weiteren physikalischen, physikochemischen beziehungsweise elektrooptischen Parameter der erfindungsgemäßen Verbindungen sind für den Einsatz der Verbindungen in flüssigkristallinen Medien von Vorteil. Die Verbindungen bzw. flüssigkristalline Medien, die diese Verbindungen enthalten, weisen insbesondere eine ausreichende Breite der nematischen Phase und eine gute Tieftemperatur- und Langzeitstabilität sowie ausreichend hohe Klärpunkte auf.

Es ist bevorzugt, dass Y für Sauerstoff, Schwefel, CH₂, CCl₂ oder CF₂ steht; insbesondere ist Y ein Sauerstoffatom oder CF₂.

A¹ und A² sind bevorzugt und unabhängig voneinander ein gegebenenfalls substituiertes 1,4-Phenylen, ein gegebenenfalls substituiertes 1,4-Cyclohexylen, worin -CH₂- ein- oder zweimal durch -O- ersetzt sein kann, oder ein gegebenenfalls substituiertes 1,4-Cyclohexenylen. Wenn n oder m 2 oder 3 sind, können die Ringe A¹ beziehungsweise A² die gleichen oder unterschiedliche Bedeutungen annehmen.

Besonders bevorzugt sind A¹ und A² unabhängig voneinander

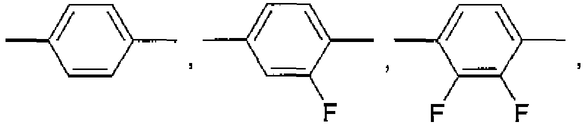

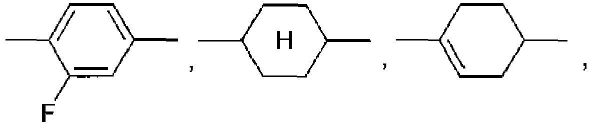

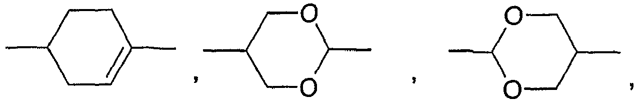

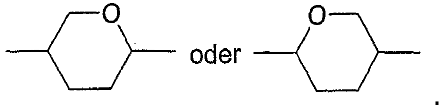

Ganz besonders bevorzugt sind A¹ und A² 1,4-Cyclohexylenringe und/oder gegebenenfalls mit Fluor substituierte 1,4-Phenylenringe.

Bevorzugt sind Z¹ und Z² unabhängig voneinander eine Einfachbindung, -CH₂O-, -OCH₂-, -CF₂O-, -OCF₂-, -CF₂CF₂-, -CH=CH-, -CF=CH-, -CH=CF- oder -CF=CF-, besonders bevorzugt unabhängig voneinander eine Einfachbindung, -CF₂O-, -OCF₂-, -CF₂CF₂-, -CH=CH-, -CF=CH-, -CH=CF- oder -CF=CF-. Ganz besonders bevorzugt sind Z¹ und Z² unabhängig voneinander eine Einfachbindung, -CF₂O-, -OCF₂-, -CF₂CF₂- oder -CF=CF-.

Sofern R¹ und R² in Formel I jeweils unabhängig voneinander einen Alkanylrest und/oder einen Alkoxyrest (Alkyloxyrest) mit 1 bis 15 C-Atomen darstellen, sind diese geradkettig oder verzweigt. Vorzugsweise ist jeder dieser Reste geradkettig, hat 1, 2, 3, 4, 5, 6 oder 7 C-Atome und ist demnach vorzugsweise Methyl, Ethyl, Propyl, Butyl, Pentyl, Hexyl, Heptyl, Methoxy, Ethoxy, Propoxy, Butoxy, Pentoxy, Hexoxy oder Heptoxy.

R¹ und R² in Formel I können jeweils unabhängig voneinander auch ein Oxaalkylrest sein, d.h. ein Alkanylrest, in dem mindestens eine der nichtterminalen CH₂-Gruppen des Alkanylrests durch -O- ersetzt ist, vorzugsweise geradkettiges 2-Oxapropyl (= Methoxymethyl), 2- (= Ethoxymethyl) oder 3-Oxabutyl (= Methoxyethyl), 2-, 3- oder 4-Oxapentyl, 2-, 3-, 4- oder 5-Oxahexyl, 2-, 3-, 4-, 5- oder 6-Oxaheptyl. In entsprechender Weise können R¹ und R² in Formel I auch unabhängig voneinander Thioalkanyl- bzw. Sulfonalkanylreste sein, d.h. Alkanylreste, in denen eine CH₂-Gruppe durch -S- beziehungsweise -SO₂- ersetzt ist.

R¹ und R² in Formel I können ferner jeweils unabhängig voneinander ein Alkenylrest mit 2 bis 15 C-Atomen sein, der geradkettig oder verzweigt ist und wenigstens eine C-C-Doppelbindung aufweist. Vorzugsweise ist er geradkettig und hat 2 bis 7 C-Atome. Er ist demnach vorzugsweise Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl. Sind die beiden C-Atome der C-C-Doppelbindung substituiert, kann der Alkenylrest als E- und/oder Z-Isomer (trans/cis) vorliegen. Im allgemeinen sind die jeweiligen E-Isomeren bevorzugt.

In gleicher Weise wie bei einem Alkanylrest kann auch bei einem Alkenylrest wenigstens eine der CH₂-Gruppen durch Sauerstoff, Schwefel oder -SO₂- ersetzt sein. Bei dem Ersatz durch -O- liegt dann ein Alkenyloxyrest (mit entständigem Sauerstoff) beziehungsweise ein Oxaalkenylrest (mit nicht-terminalem Sauerstoff) vor.

R¹ und R² in Formel I können unabhängig voneinander auch ein Alkinylrest mit 2 bis 15 C-Atomen sein, der geradkettig oder verzweigt ist und wenigstens eine C-C-Dreifachbindung aufweist.

R¹ und R² in Formel I können jeweils unabhängig voneinander ein Alkanylrest mit 1 bis 15 C-Atomen sein, in dem eine CH₂-Gruppe durch -O- und eine durch -CO- ersetzt ist, wobei diese bevorzugt benachbart sind. Somit beinhaltet dieser eine Acyloxygruppe -CO-O- oder eine Oxycarbonylgruppe -O-CO-. Vorzugsweise ist dieser Rest geradkettig und hat 2 bis 6 C-Atome. Dabei sind die folgenden dieser Reste bevorzugt: Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 2-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)- propyl oder 4-(Methoxycarbonyl)butyl. Ferner kann ein Alkanylrest auch eine -O-CO-O-Einheit aufweisen. Auch der Ersatz einer CH₂-Gruppe durch lediglich eine -CO-Gruppe (Carbonylfunktion) ist möglich.

R¹ und R² in Formel I können jeweils unabhängig voneinander ein Alkenylrest mit 2 bis 15 C-Atomen sein, in dem eine CH₂-Gruppe bevorzugt in Nachbarschaft zu einer unsubstituierten oder substituierten -C=C-Einheit durch -CO-, -CO-O-, -O-CO- oder -O-CO-O- ersetzt ist, wobei dieser Rest geradkettig oder verzweigt sein kann. Vorzugsweise ist der Rest geradkettig und hat 4 bis 13 C-Atome. Besonders bevorzugt sind dabei Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxy-heptyl, 8-Acryloyloxyoctyl, 9-Acryloyloxynonyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl oder 8-Methacryloyloxyoctyl. Entsprechend kann auch in einem Alkinylrest in Nachbarschaft zu einer substituierten -C≡C-Einheit eine CH₂-Gruppe durch -CO-, -CO-O-, -O-CO- oder -O-CO-O- ersetzt sein.

R¹ und R² in Formel I können jeweils unabhängig voneinander ein einfach durch -CN oder -CF₃ substituierter Alkanylrest oder Alkoxyrest mit 1 bis 15 C-Atomen oder ein Alkenylrest oder Alkinylrest mit 2 bis 15 C-Atomen sein, wobei diese vorzugsweise geradkettig sind. Die Substitution durch -CN oder -CF₃ ist in beliebiger Position möglich.

R¹ und R² in Formel I können jeweils unabhängig voneinander ein Alkanylrest sein, in dem zwei oder mehr CH₂-Gruppen durch -O- und/oder -CO-O- ersetzt sind, wobei dieser geradkettig oder verzweigt sein kann. Vorzugsweise ist er verzweigt und hat 3 bis 12 C-Atome.

R¹ und R² in Formel I können jeweils unabhängig voneinander ein einfach oder mehrfach mit F, Cl, Br und/oder I substituierter Alkanylrest oder Alkoxyrest mit 1 bis 15 C-Atomen oder Alkenylrest oder Alkinylrest mit 2 bis 15 C-Atomen sein, wobei diese Reste vorzugsweise geradkettig sind und Halogen vorzugsweise -F und/oder -Cl ist. Bei Mehrfachsubstitution ist Halogen vorzugsweise -F. Die resultierenden Reste schließen auch perfluorierte Reste wie -CF₃ ein. Bei Einfachsubstitution kann der Fluor- oder Chlorsubstituent in beliebiger Position sein, vorzugsweise ist er in ω-Position.

R¹ und R² in Formel I können auch jeweils unabhängig voneinander -F, -Cl, -Br, -I, -CN, -SCN, -NCS oder -SF₅ sein.

Besonders bevorzugt sind R¹ und R² unabhängig voneinander in der allgemeinen Formel I Wasserstoff oder ein Alkanylrest, Alkoxyrest oder Alkenylrest mit 1 bis 7 bzw. 2 bis 7 C-Atomen, wobei jeder dieser Reste bevorzugt unsubstituiert oder mit Halogen einfach oder mehrfach substituiert ist.

Halogen bedeutet im Zusammenhang der vorliegenden Erfindung Fluor, Chlor, Brom und Iod.

Im Zusammenhang mit der vorliegenden Erfindung bedeutet der Ausdruck "Alkyl" - sofern er nicht an anderer Stelle dieser Beschreibung oder in den Ansprüchen abweichend definiert ist - einen geradkettigen oder verzweigten aliphatischen Kohlenwasserstoffrest mit 1 bis 15 (d.h. 1, 2, 3, 4, 5, 6, 7, 8, 9, 10, 11, 12, 13, 14 oder 15) Kohlenstoffatomen. Sofern es sich bei diesem Alkylrest um einen gesättigten Rest handelt, wird er auch als "Alkanyl" bezeichnet.

Bevorzugte Verbindungen der allgemeinen Formel I weisen insgesamt keine, eine, zwei oder drei Einheiten -Z¹-A¹- und/oder -Z²-A²- auf, das heißt m + n = 0, 1, 2 oder 3 mit m und n jeweils 0, 1, 2 oder 3. Sind zwei oder drei Einheiten -Z¹-A¹- und/oder -Z²-A²- vorhanden, können sie an nur eine Molekülseite (d.h. m = 2 oder 3 und n = 0 oder n = 2 oder 3 und m = 0) oder auch an beide Molekülseiten gebunden sein. Besonders bevorzugt ist m + n = 0, 1 oder 2.

Bevorzugte Verbindungen der Formel I, für die gilt: m + n = 0, werden durch die folgende Formel dargestellt: 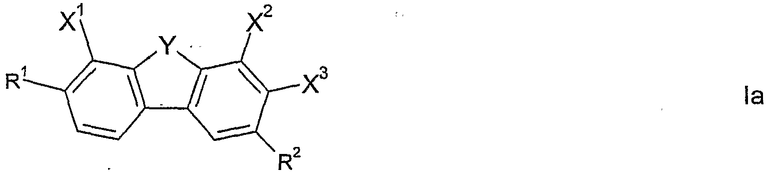 worin R¹, R², X¹, X², X³ und Y die gleichen sowie die gleichen bevorzugten Bedeutungen wie für Formel I oben definiert besitzen. Insbesondere steht Y für ein Sauerstoffatom oder eine CF₂-Gruppe.

Bevorzugte Verbindungen der Formel I, für die gilt: m + n = 1, werden durch die folgenden Formeln dargestellt: 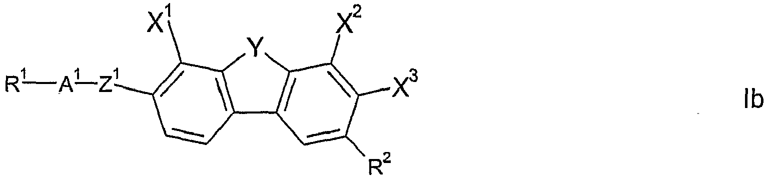 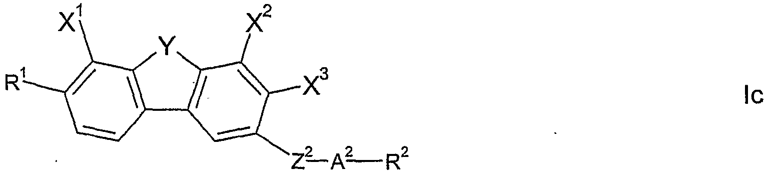 worin R¹, R², A¹, A², X¹, X², X³, Z¹, Z² und Y die gleichen sowie die gleichen bevorzugten Bedeutungen wie für Formel I oben definiert besitzen. Insbesondere steht Y für ein Sauerstoffatom oder eine CF₂-Gruppe.

Bevorzugte Verbindungen der Formel I, für die gilt: m + n = 2, werden durch die folgenden Formeln dargestellt: 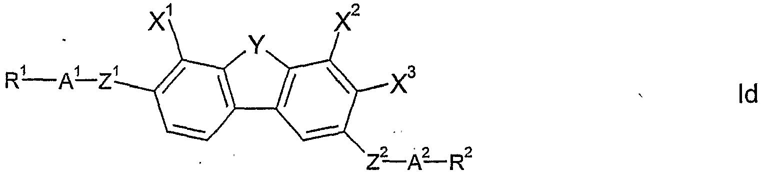 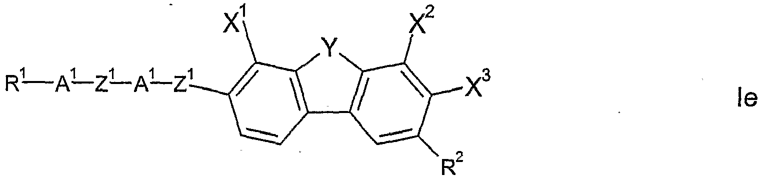 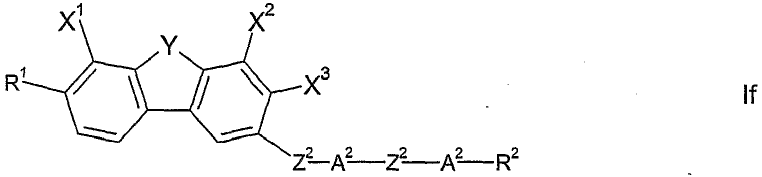 worin R¹, R², A¹, A², X¹, X², X³, Z¹, Z² und Y die gleichen sowie die gleichen bevorzugten Bedeutungen wie für Formel I oben definiert besitzen. Soweit A¹, A², Z¹ beziehungsweise Z² in den Formeln Ie und If zweifach vorkommen, können sie jeweils die gleiche oder verschiedene Bedeutungen aufweisen. Insbesondere steht Y für ein Sauerstoffatom oder eine CF₂-Gruppe.

Bevorzugte Verbindungen der Formel I, für die gilt: m + n = 3, werden durch die folgenden Formeln dargestellt: 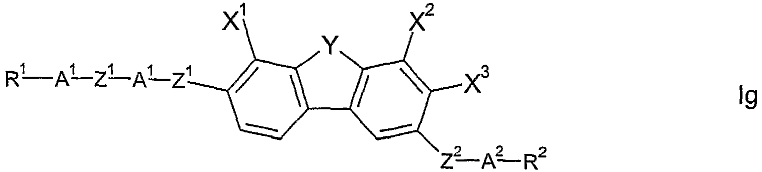 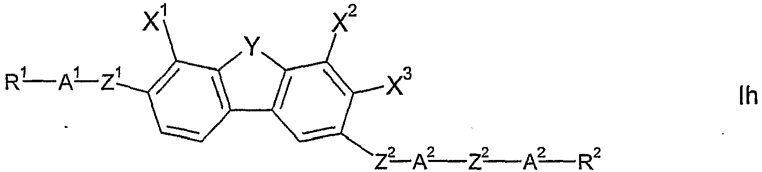 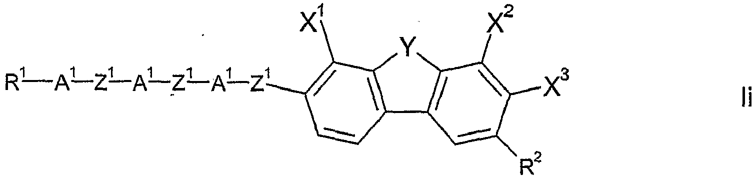 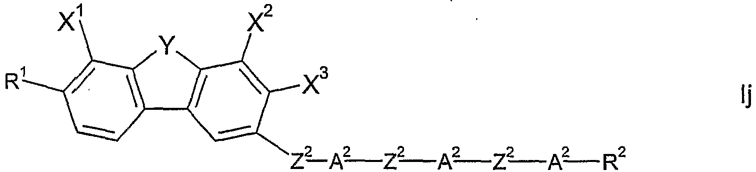 worin R¹, R², A¹, A², X¹, X², X³, Z¹, Z² und Y die gleichen sowie die gleichen bevorzugten Bedeutungen wie für Formel I oben definiert besitzen. Soweit A¹, A², Z¹ beziehungsweise Z² in den Formeln Ig bis Ij mehr als einmal vorkommen, können sie jeweils die gleiche oder verschiedene Bedeutungen aufweisen. Insbesondere steht Y für ein Sauerstoffatom oder eine CF₂-Gruppe.

Besonders bevorzugt sind erfindungsgemäße Verbindungen der Formeln Ia, Ib, Ic und Id, insbesondere der Formeln Ia, Ib und Id.

Ganz besonders bevorzugte Verbindungen der Formel Ia sind die folgenden: 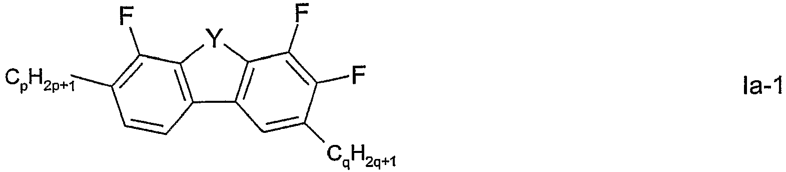 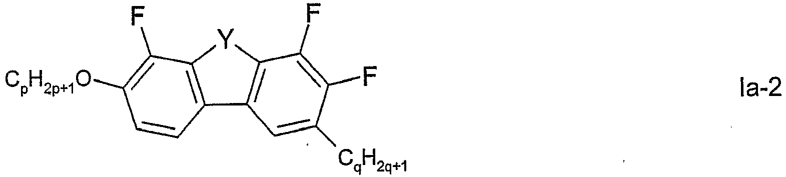 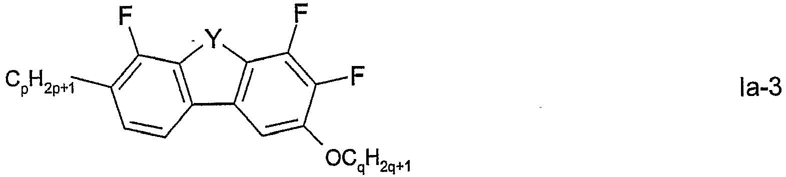 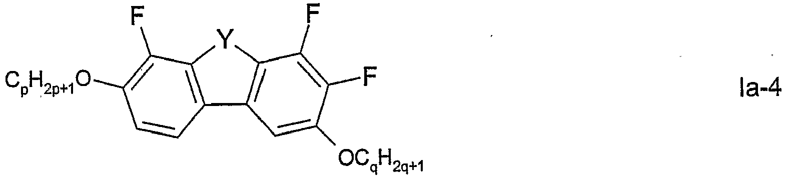 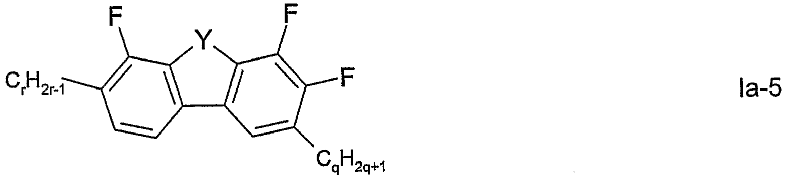 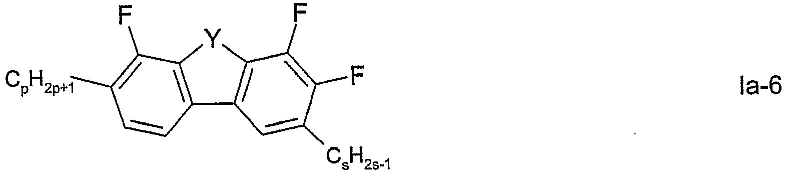 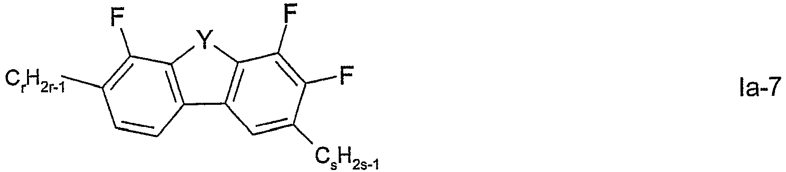 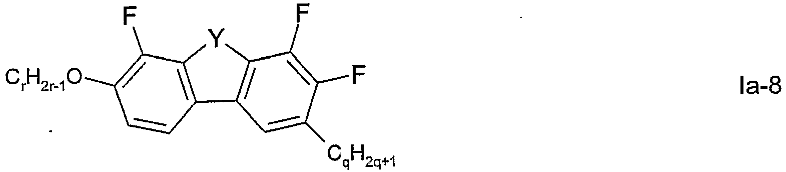 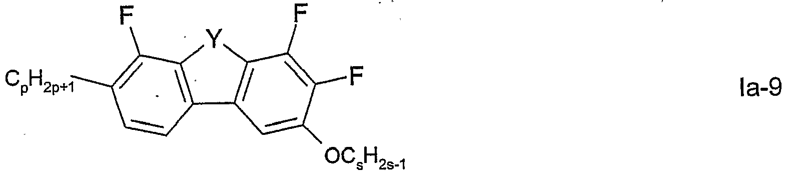 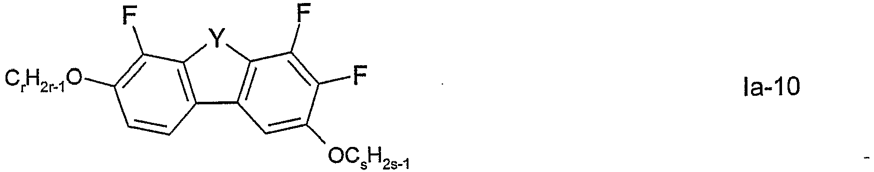 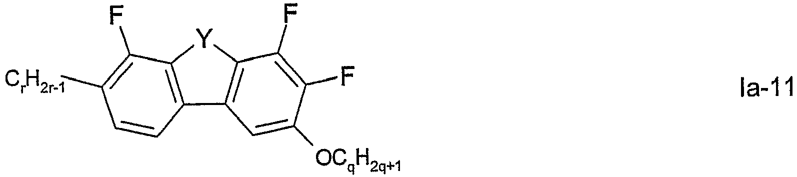 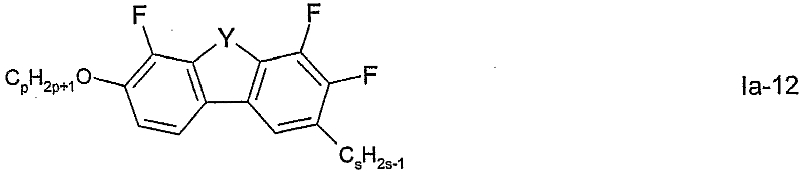 worin p und q jeweils unabhängig voneinander 1, 2, 3, 4, 5, 6, 7 oder 8 sind und r und s jeweils unabhängig voneinander 2, 3, 4, 5, 6, 7 oder 8 sind, wobei die jeweiligen Reste bevorzugt geradkettig sind und ein bis vier der Wasserstoffatome in jedem der Reste durch Fluor ersetzt sein können und Y für ein Sauerstoffatom oder eine CF₂-Gruppe steht. Unter den Verbindungen der Formeln la-1 bis Ia-12 sind jene der Formeln Ia-1 bis la-7 noch weiter bevorzugt, insbesondere Verbindungen der Formel Ia-1.

Unter den bevorzugten erfindungsgemäßen Verbindungen der Formel Ib sind die folgenden besonders bevorzugt: 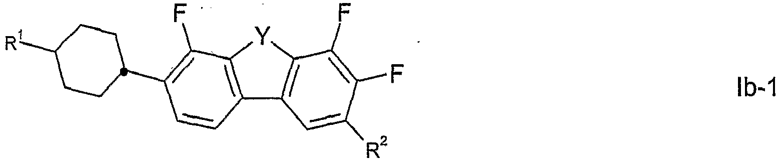 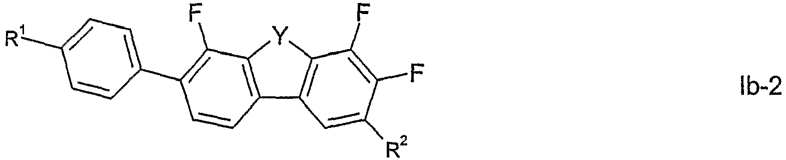 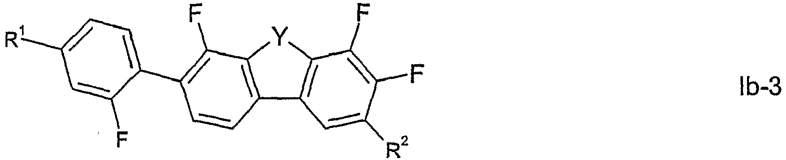 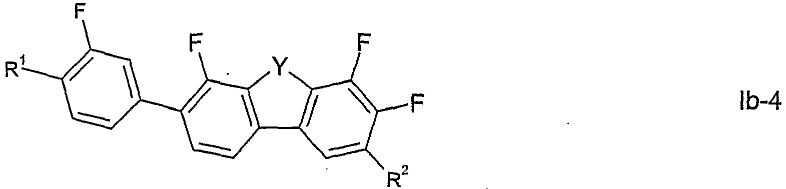 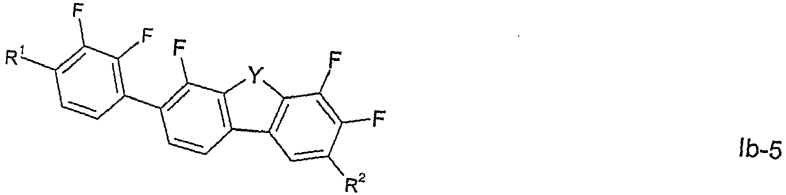 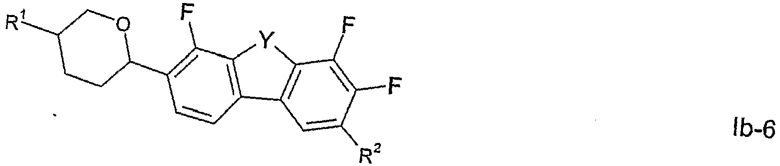 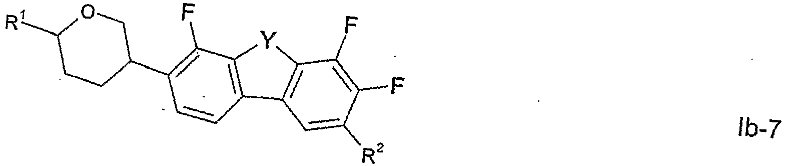 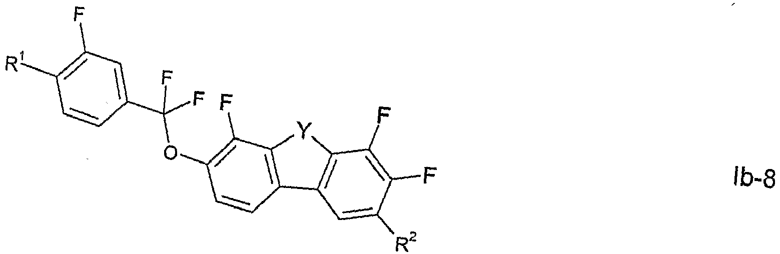 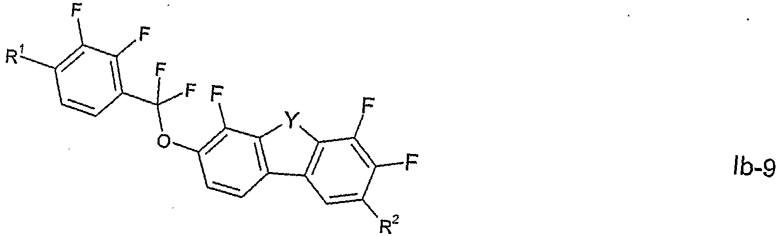 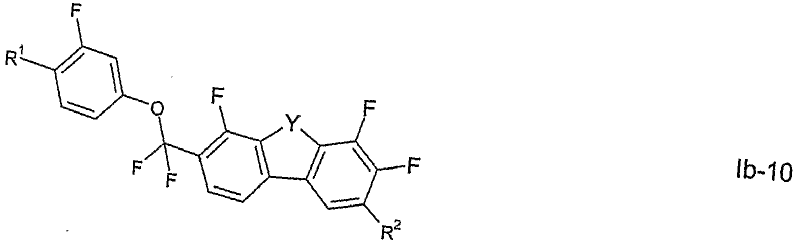 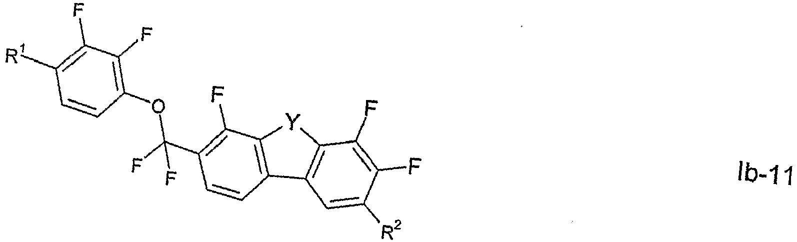 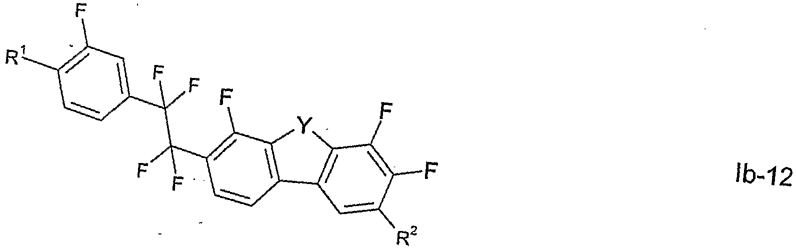 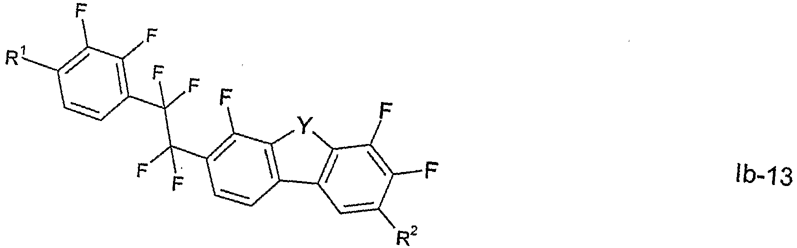 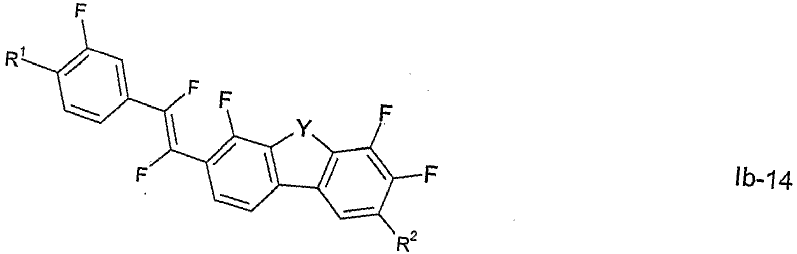 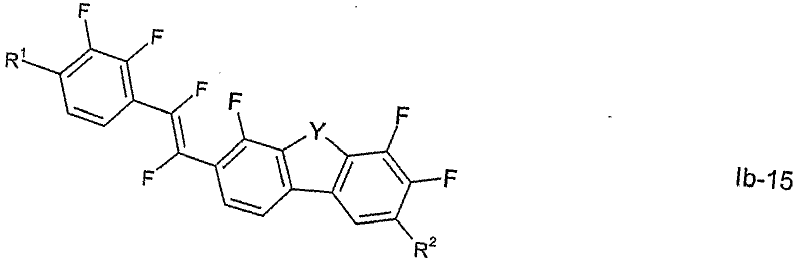 worin R¹, R² und Y die gleichen sowie die gleichen bevorzugten Bedeutungen wie für Formel I oben definiert besitzen. Ganz besonders bevorzugt ist R¹ ein Alkanylrest, Alkoxyrest oder Alkenylrest mit 1 bis 7 bzw. 2 bis 7 Kohlenstoffatomen, R² Wasserstoff oder ein Alkanylrest, Alkoxyrest oder Alkenylrest mit 1 bis 7 bzw. 2 bis 7 Kohlenstoffatomen und Y ein Sauerstoffatom oder eine CF₂-Gruppe.

Unter den bevorzugten erfindungsgemäßen Verbindungen der Formel Id sind die folgenden besonders bevorzugt: 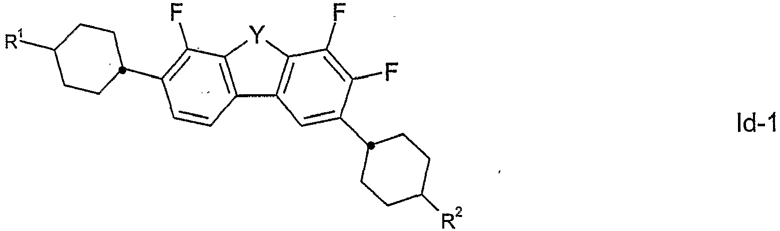 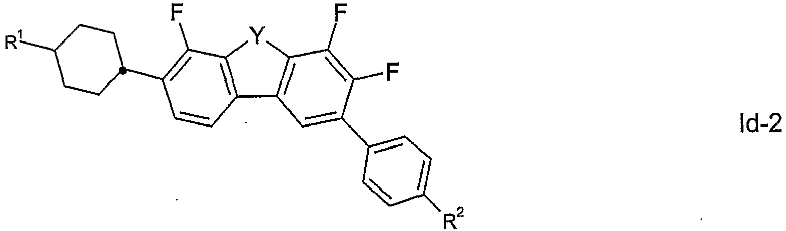 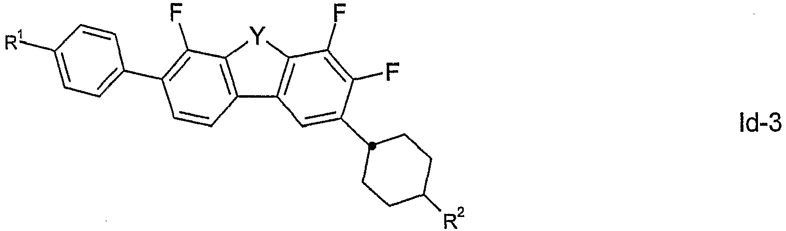 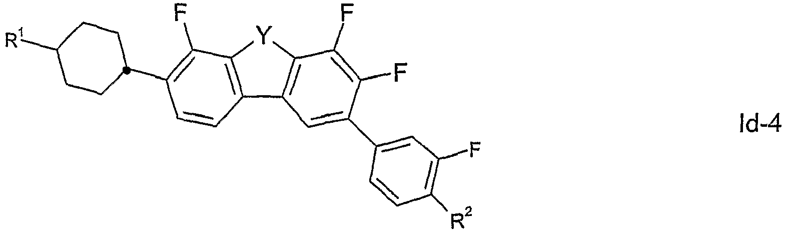 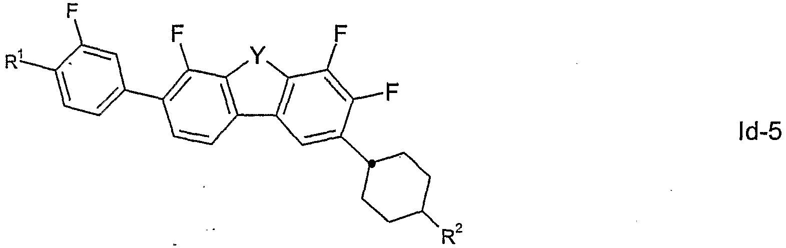 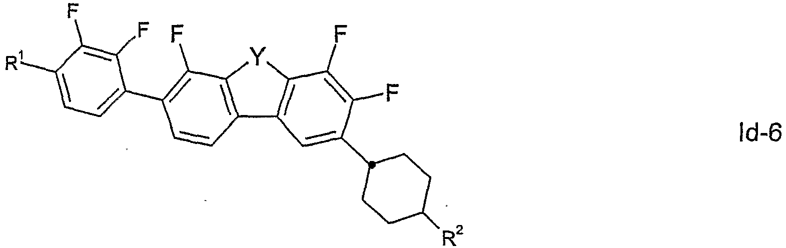 worin R¹, R² und Y die gleichen sowie die gleichen bevorzugten Bedeutungen wie für Formel I oben definiert besitzen. Ganz besonders bevorzugt ist R¹ ein Alkanylrest, Alkoxyrest oder Alkenylrest mit 1 bis 7 bzw. 2 bis 7 Kohlenstoffatomen, R² Wasserstoff oder ein Alkanylrest, Alkoxyrest oder Alkenylrest mit 1 bis 7 bzw. 2 bis 7 Kohlenstoffatomen und Y ein Sauerstoffatom oder eine CF₂-Gruppe. Unter den Verbindungen der Formeln Id-1 bis Id-6 sind Verbindungen der Formel Id-1 am meisten bevorzugt.

Sofern Reste oder Substituenten der erfindungsgemäßen Verbindungen beziehungsweise die erfindungsgemäßen Verbindungen selbst als optisch aktive oder stereoisomere Reste, Substituenten beziehungsweise Verbindungen vorliegen, weil sie beispielsweise ein asymmetrisches Zentrum aufweisen, so sind diese von der vorliegenden Erfindung mit umfasst. Dabei ist es selbstverständlich, dass die erfindungsgemäßen Verbindungen der allgemeinen Formel I in isomerenreiner Form, zum Beispiel als reine Enantiomeren, Diastereomeren, E- beziehungsweise Z-Isomeren, trans- beziehungsweise cis-Isomeren, oder als Gemisch mehrerer Isomeren in jedem beliebigen Verhältnis, zum Beispiel als Racemat, E-/Z-Isomerengemisch oder als cis/trans-Isomerengemisch, vorliegen können.

Die Verbindungen der allgemeinen Formel I werden nach an sich bekannten Methoden dargestellt, wie sie in der Literatur (z.B. in den Standardwerken wie Houben-Weyl, Methoden der organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Ausgangsstoffe können gegebenenfalls auch in situ gebildet werden, derart, dass man sie aus dem Reaktionsgemisch nicht isoliert, sondern sofort weiter zu den Verbindungen der allgemeinen Formel I umsetzt.

Die Synthesen verschiedener erfindungsgemäßer Verbindungen der allgemeinen Formel I werden in den Beispielen exemplarisch beschrieben. Die Ausgangssubstanzen sind nach allgemein zugänglichen Literaturvorschriften oder käuflich zu erhalten. Die beschriebenen Reaktionen sind als literaturbekannt anzusehen.

Die beispielhafte Synthese eines erfindungsgemäßen Dibenzofurans (Y = O, X¹, X², X³ = F, m, n = 0 und R¹, R² = n-Propyl) ist im folgenden dargestellt. Die Synthese kann durch die Wahl geeigneter Ausgangsprodukte an die jeweils gewünschten Verbindungen der allgemeinen Formel I angepasst werden. Die einzelnen Syntheseschritte sind ebenfalls als literaturbekannt anzusehen.

### Synthese der Boronsäure 4:

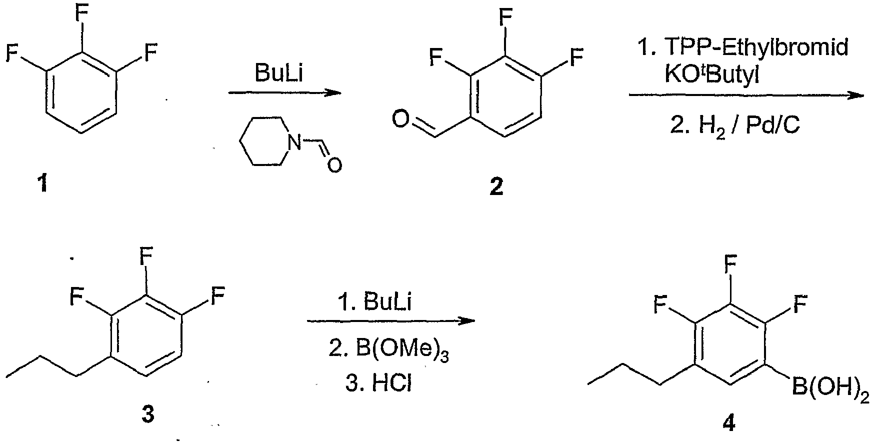

Trifluorbenzol **1** wird mit Butyllithium und N-Formylpiperidin in den Aldehyd **2** übergeführt. Nach der anschließenden Wittig-Reaktion erfolgt eine Hydrierung am Palladium-Katalysator. Das Trifluorpropylbenzol **3** wird unter Standardbedingungen in die Boronsäure **4** umgewandelt.

### Synthese des Silylethers 8:

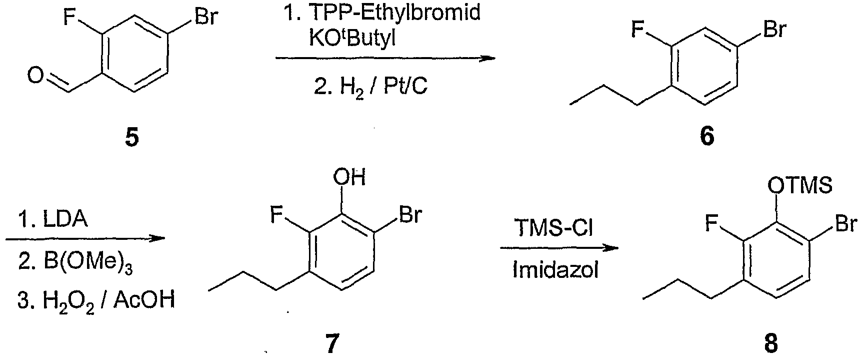

Der Aldehyd **5** wird mittels einer Wittig-Reaktion und anschließender Hydrierung am Platin-Katalysator in das Bromfluorpropylbenzol **6** übergeführt. Der aus Lithiumdiisopropylamid (LDA) und Trimethylborat erhaltene Boronsäureester wird analog zu einer Hydroborierung in das Phenol 7 umgewandelt. Die OH-Funktion wird mit Trimethylsilylchlorid (TMS-Cl) und Imidazol zum Silylether **8** umgesetzt.

### Aufbau des Dibenzofurans:

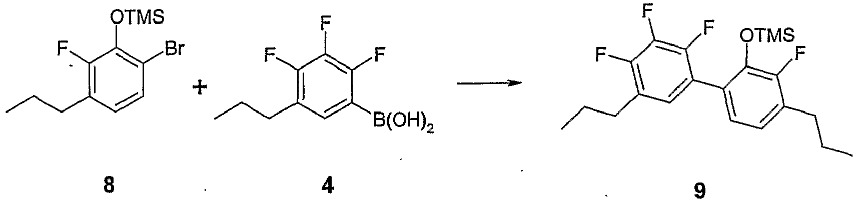

Der Silylether **8** wird unter Stickstoff in Toluol gelöst, mit Natriumcarbonat, Wasser und Tetrakis(triphenylphosphin)palladium versetzt. In der Siedehitze wird dem Reaktionsgemisch langsam eine Lösung der Boronsäure **4** in absolutem Ethanol zugesetzt. Nach Kochen unter Rückfluss und Abkühlen werden die Phasen getrennt. Die wässrige Phase wird mit MTB-Ether extrahiert. Die organische Phase wird über Natriumsulfat getrocknet und eingeengt. Der Rückstand wird an Kieselgel chromatograhiert.

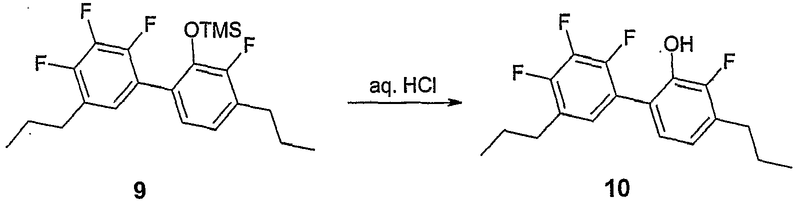

Der Silylether **9** wird in THF gelöst und mit verd. HCl versetzt. Die Lösung wird bis zum vollständigen Umsatz bei Raumtemperatur gerührt. Anschließend wird das Lösungsmittel entfernt und der Rückstand wird in verd. HCl-Lösung und MTB-Ether aufgenommen. Die wässrige Phase wird dreimal mit MTB-Ether extrahiert. Die organische Phase wird getrocknet, eingeengt und über Kieselgel gegeben.

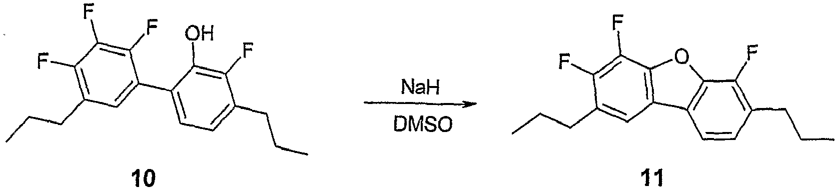

Das Phenol **10** wird unter Stickstoff in DMSO gelöst, mit einer 60 %igen Natriumhydrid-Mineralölsuspension versetzt und auf 120 °C erwärmt. Nach 5 Stunden bei dieser Temperatur wird der abgekühlte Ansatz in verd. HCl-Lösung gegeben. Die wässrige Phase wird mit MTB-Ether extrahiert. Die organische Phase wird über Natriumsulfat getrocknet, eingeengt und an Kieselgel chromatographiert.

Die beispielhafte Synthese eines erfindungsgemäßen Fluorenderivates (Y = CF₂, X¹, X², X³ = F, m, n = 0 und R¹ = Ethoxy, R² = n-Propyl) ist im folgenden dargestellt. Die Synthese kann durch die Wahl geeigneter Ausgangsprodukte an die jeweils gewünschten Verbindungen der allgemeinen Formel I angepasst werden. Die einzelnen Syntheseschritte sind ebenfalls als literaturbekannt anzusehen.

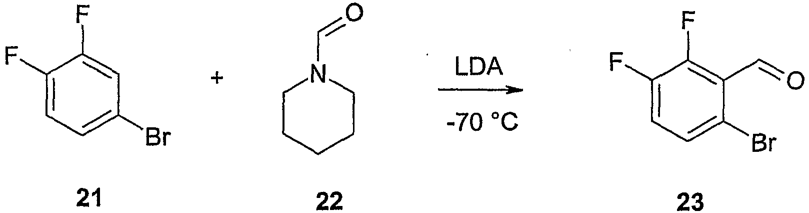

Unter Stickstoff und bei -70 °C wird eine Lösung des Aromaten **21** in THF mit einer 2 M Lösung von Lithiumdiisopropylamid in Cyclohexan/Ethyl-benzol/THF versetzt. Dem Ansatz wird N-Formylpiperidin **22** hinzugefügt. Der Ansatz wird bei 0 °C hydrolysiert und mit Salzsäure angesäuert. Nach der Aufarbeitung erhält man erhält den Aldehyd **23.**

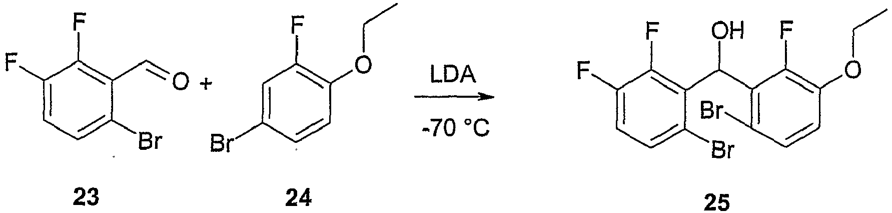

Unter Stickstoff und bei 70 °C wird eine Lösung des Aromaten **24** in THF mit einer 2 M Lösung von Lithiumdiisopropylamid in Cyclohexan/Ethyl-benzol/THF versetzt. Dem Ansatz wird der Aldehyd **23** gelöst in THF hinzugefügt. Der Ansatz wird hydrolysiert und mit Salzsäure angesäuert. Nach der Aufarbeitung erhält man erhält das Diphenylmethanol **25**.

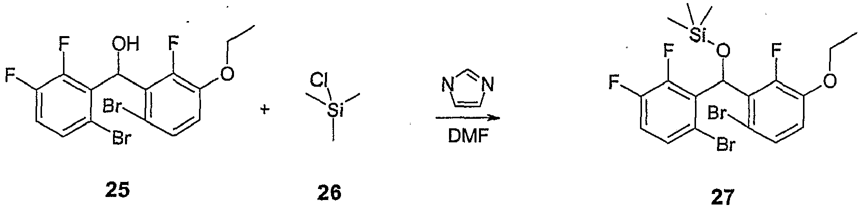

Unter Stickstoff werden Imidazol und das Diphenylmethanol **25** in DMF gelöst, mit Trimethylsilylchlorid **26** versetzt und bei Raumtemperatur über Nacht gerührt. Nach der Aufarbeitung erhält man erhält den Silylether **27**.

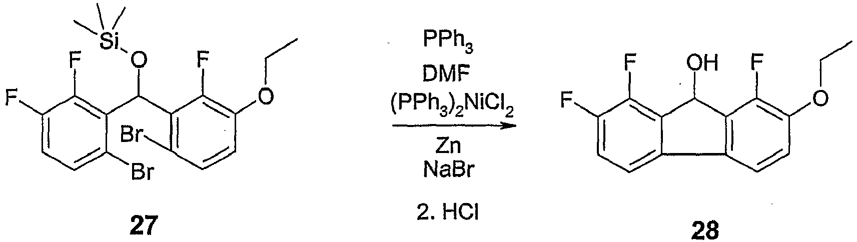

Unter Stickstoff werden Zink, Bis-triphenylphosphin-Nickel(II)chlorid, Natriumbromid und Triphenylphosphin mit DMF versetzt und auf 80 °C erwärmt. Nach 30 Minuten wird der Silylether **27** gelöst in DMF tropfenweise in den Ansatz gegeben. Nach der Aufarbeitung erhält man das Fluorenol **28**.

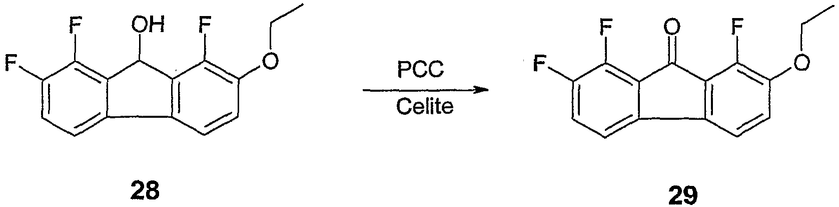

Unter Stickstoff werden Pyridiniumchlorochromat (PCC) in eine Suspension von Celite in Dichlormethan gegeben. Anschließend wird eine Lösung des Fluorenols 28 in Dichlormethan in die Suspension gegeben. Der Ansatz wird über Nacht bei Raumtemperatur gerührt. Nach der Aufarbeitung erhält man das Fluorenon **29**.

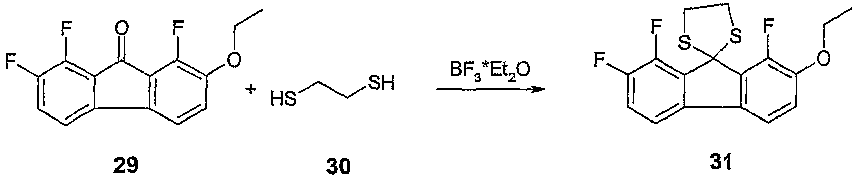

Unter Stickstoff wird bei -10 °C eine Lösung des Fluorenons **29** und Ethandithiol **30** in Dichlormethan mit Bortrifluorid-Diethylether-Komplex versetzt. Der Ansatz taut über Nacht auf und wird vorsichtig auf ges. Natriumhydrogencarbonat-Lösung gegeben. Der pH Wert wird auf 8 eingestellt. Nach der Aufarbeitung erhält man das Thioketal **31.**

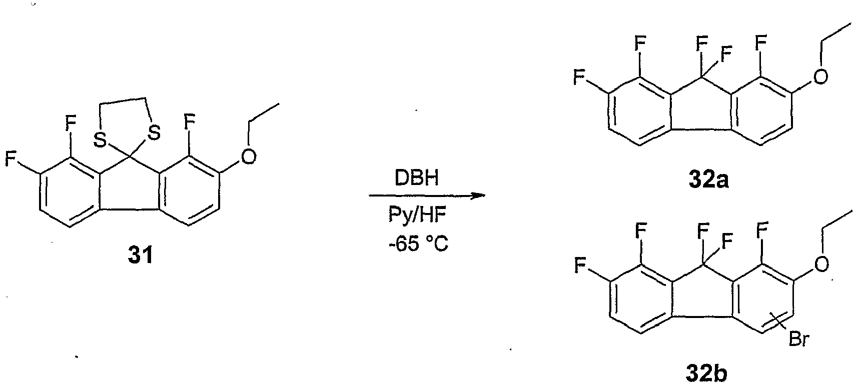

Unter Stickstoff und bei einer Temperatur von unter -65 °C wird eine Suspension von 1,3-Dibrom-5,5-dimethylhydantoin in Dichlormethan und einer 65 %igen Lösung von HF in Pyridin mit einer Lösung des Thioketals **31** in Dichlormethan versetzt. Nach 5 Stunden wird die Kühlung entfernt und der Ansatz rührt über Nacht. Anschließend wird der Ansatz auf eisgekühlte 1 N Natronlauge, die mit 19 %iger NatriumhydrogensulfitLösung versetzt worden ist, gegeben. Der pH wird auf 8 eingestellt. Nach der Aufarbeitung erhält man ein Gemisch der Fluorene **32a** und **32b**.

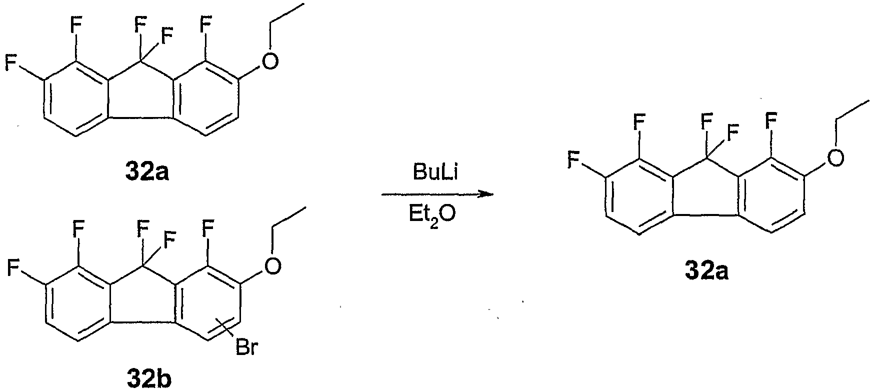

Das Fluorengemisch wird unter Stickstoff in Diethylether gelöst und bei -70 °C mit einer 15 %igen Lösung von Butyllithium in n-Hexan versetzt. Nach 1 Stunde wird eine 1:1-THF/Wasser-Mischung der Lösung zugesetzt und der Ansatz auf Raumtemperatur erwärmt. Nach der Aufarbeitung erhält man das Fluoren 32a.

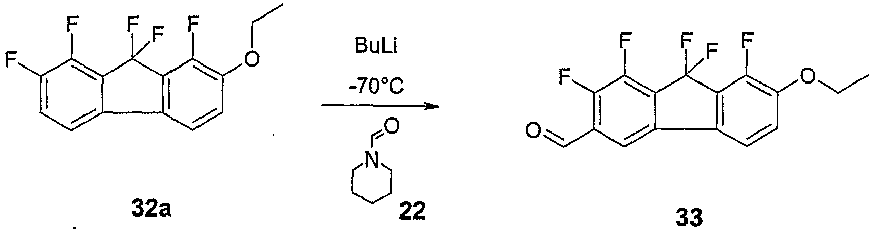

Unter Stickstoff und bei -70 °C wird eine Lösung des Fluorens **32a** in THF mit einer 15 %igen Lösung von Butyllithium in n-Hexan versetzt. Dem Ansatz wird N-Formylpiperidin **22** hinzugefügt. Der Ansatz wird hydrolysiert und mit Salzsäure angesäuert. Nach der Aufarbeitung erhält man den Aldehyd **33**.

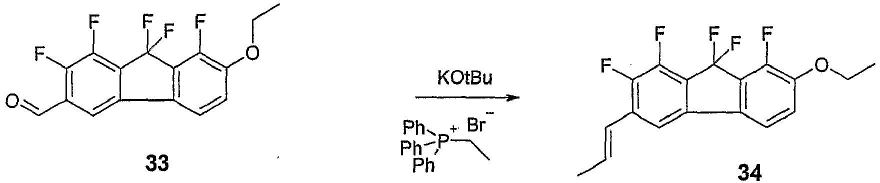

Unter Stickstoff wird eine Suspension von Ethyl-tris-triphenylphosphoniumbromid in THF bei 0 °C mit einer Lösung von Kalium-*tert-*Butylat in THF versetzt. Nach 1 Stunde wird der Aldehyd **33** gelöst in THF langsam zugegeben. Der Ansatz wird über Nacht bei Raumtemperatur gerührt und anschließend mit Wasser versetzt. Nach der Aufarbeitung erhält man das Olefin **34.**

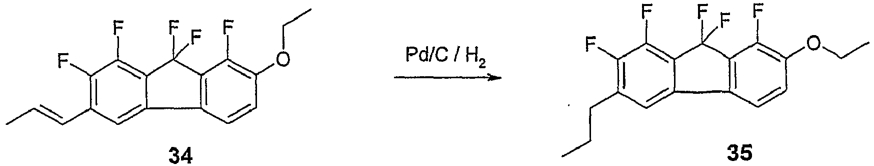

Das Olefin **34** wird in THF gelöst und am Palladiumkatalysator hydriert. Die Hydrierlösung wird eingeengt. Nach der Aufarbeitung erhält man das Pentafluorfluoren **35.**

Die dargestellten Reaktionsschemata sind nur als beispielhaft aufzufassen. Der Fachmann kann entsprechende Variationen der vorgestellten Synthesen vornehmen sowie auch andere geeignete Synthesewege beschreiten, um Verbindungen der Formel I zu erhalten.

Wie bereits erwähnt, können die Verbindungen der allgemeinen Formel I in flüssigkristallinen Medien verwendet werden.

Gegenstand der vorliegenden Erfindung ist daher auch ein flüssigkristallines Medium mit mindestens zwei flüssigkristallinen Verbindungen, enthaltend mindestens eine Verbindung der allgemeinen Formel I.

Gegenstand der vorliegenden Erfindung sind auch flüssigkristalline Medien enthaltend neben einer oder mehreren erfindungsgemäßen Verbindungen der Formel I als weitere Bestandteile 2 bis 40, vorzugsweise 4 bis 30 Komponenten. Besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, 1,3-Dioxane, 2,5-Tetrahydro-pyrane, Phenyl- oder Cyclohexylbenzoate, Cyclohexancarbonsäurephenyl-oder-cyclohexylester, Phenyl- oder Cyclohexylester der Cyclohexylbenzoe-säure, Phenyl- oder Cyclohexylester der Cyclohexylcyclohexancarbon-säure, Cyclohexylphenylester der Benzoesäure, der Cyclohexancarbon-säure, bzw. der Cyclohexylcyclohexancarbonsäure, Phenylcyclohexane, Cyclohexylbiphenyle, Phenylcyclohexylcyclohexane, Cyclohexylcyclo-hexane, Cyclohexylcyclohexylcyclohexene, 1,4-Biscyclohexylbenzole, 4',4'-Biscyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenylcyclohexyl)ethane, 1-Cyclohexyl-2-biphenylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch einfach oder mehrfach fluoriert sein.

Die wichtigsten als weitere Bestandteile erfindungsgemäßer Medien in Frage kommenden Verbindungen lassen sich durch die Formeln (II), (III), (IV), (V) und (VI) charakterisieren:

R'-L-E-R" (II)

R'-L-COO-E-R" (III)

R'-L-OOC-E-R" - (IV)

R'-L-CH₂CH₂-E-R" (V)

R'-L-CF₂O-E-R" (VI)

In den Formeln (II), (III), (IV), (V) und (VI) bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -Thp-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl, Thp Tetrahydropyran-2,5-diyl und G 2-(trans-1,4-Cyclohexyl)-ethyl, Pyrimidin-2,5-diyl, Pyridin-2,5-diyl, 1,3-Dioxan-2,5-diyl oder Tetrahydropyran-2,5-diyl bedeuten.

Vorzugsweise ist einer der Reste L und E Cyc oder Phe. E ist vorzugsweise Cyc, Phe oder Phe-Cyc. Vorzugsweise enthalten die erfindungs-gemäßen Medien eine oder mehrere Komponenten ausgewählt aus den Verbindungen der Formeln (II), (III), (IV), (V) und (VI), worin L und E ausgewählt sind aus der Gruppe Cyc und Phe und gleichzeitig eine oder mehrere Komponenten ausgewählt aus den Verbindungen der Formeln (II), (III), (IV), (V) und (VI), worin einer der Reste L und E ausgewählt ist aus der Gruppe Cyc und Phe und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln (II), (III), (IV), (V) und (VI), worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln (II), (III), (IV), (V) und (VI) jeweils unabhängig voneinander Alkyl, Alkenyl, Alkoxy, Alkoxyalkyl (Oxaalkyl), Alkenyloxy oder Alkanoyloxy mit bis zu 8 C-Atomen. Im folgenden wird diese kleinere Untergruppe Gruppe A genannt und die Verbindungen werden mit den Teilformeln (IIa), (IIIa), (IVa), (Va) und (VIa) bezeichnet. Bei den meisten dieser Verbindungen sind R' und R" voneinander verschieden, wobei einer dieser Reste meist Alkyl, Alkenyl, Alkoxy oder Alkoxyalkyl (Oxaalkyl) ist.

In einer anderen als Gruppe B bezeichneten kleineren Untergruppe der Verbindungen der Formeln (II), (III), (IV), (V) und (VI) bedeutet E

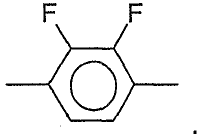

In den Verbindungen der Gruppe B, die mit den Teilformeln (IIb), (IIIb), (IVb), (Vb) und (Vlb) bezeichnet werden, haben R' und R" die bei den Verbindungen der Teilformeln (IIa) bis (VIa) angegebene Bedeutung und sind vorzugsweise Alkyl, Alkenyl, Alkoxy oder Alkoxyalkyl (Oxaalkyl).

In einer weiteren kleineren Untergruppe der Verbindungen der Formeln (II), (III), (IV), (V) und (VI) bedeutet R" -CN. Diese Untergruppe wird im folgenden als Gruppe C bezeichnet und die Verbindungen dieser Untergruppe werden entsprechend mit Teilformeln (IIc), (IIIc), (IVc), (Vc) und (Vlc) beschrieben. In den Verbindungen der Teilformeln (IIc), (IIIc), (IVc), (Vc) und (Vlc) hat R' die bei den Verbindungen der Teilformeln (IIa) bis (VIa) angegebene Bedeutung und ist vorzugsweise Alkyl, Alkenyl, Alkoxy oder Alkoxyalkyl (Oxaalkyl).

Neben den bevorzugten Verbindungen der Gruppen A, B und C sind auch andere Verbindungen der Formeln (II), (III), (IV), (V) und (VI) mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben den erfindungsgemäßen Verbindungen der allgemeinen Formel I vorzugsweise eine oder mehrere Verbindungen aus den Gruppen A, B und/oder C. Die Massenanteile der Verbindungen aus diesen Gruppen an den erfindungsgemäßen Medien betragen:
Gruppe A:
   0 bis 90 %, vorzugsweise 20 bis 90 %, insbesondere 30 bis 90 %.
Gruppe B:
   0 bis 80 %, vorzugsweise 10 bis 80 %, insbesondere 10 bis 70 %.
Gruppe C:
   0 bis 80 %, vorzugsweise 5 bis 80 %, insbesondere 5 bis 50 %.

Die erfindungsgemäßen Medien enthalten vorzugsweise 1 bis 40 %, besonders bevorzugt 5 bis 30 % an den erfindungsgemäßen Verbindungen der Formel I. Weiterhin bevorzugt sind Medien, enthaltend mehr als 40 %, insbesondere 45 bis 90 % an erfindungsgemäßen Verbindungen der Formel I. Die Medien enthalten vorzugsweise eine, zwei, drei, vier oder fünf erfindungsgemäße Verbindungen der Formel I.

Beispiele für die Verbindungen der Formeln (II), (III), (IV), (V) und (VI) sind die nachstehend aufgeführten Verbindungen: 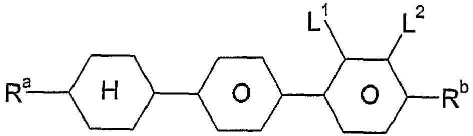 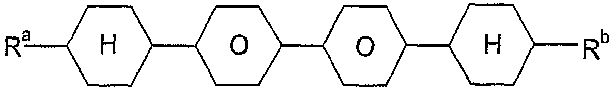 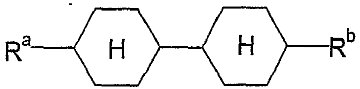 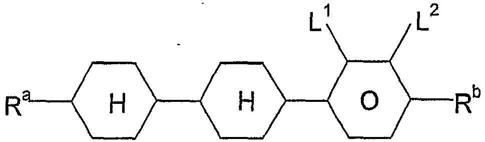 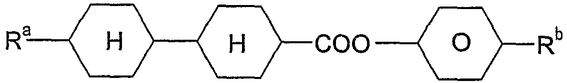 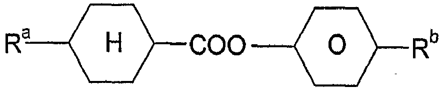 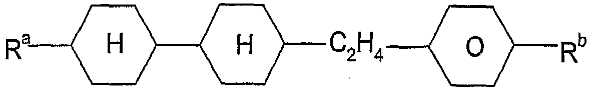 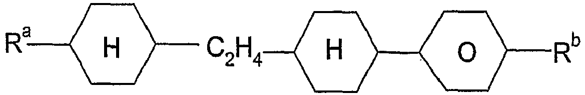 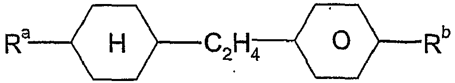 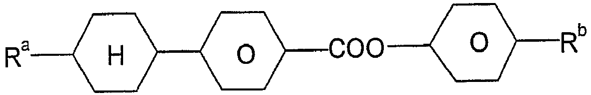 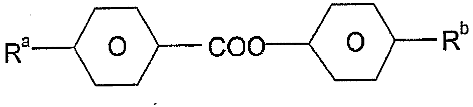 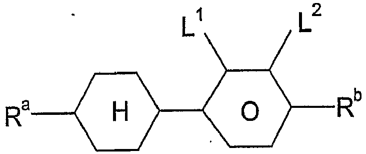 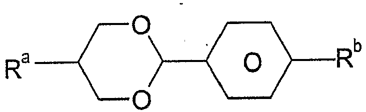 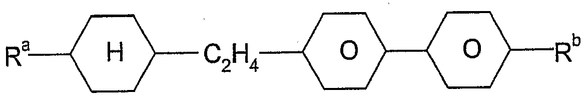 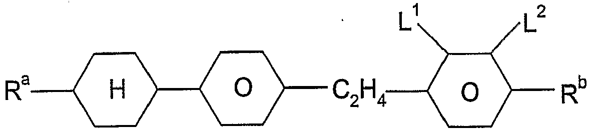 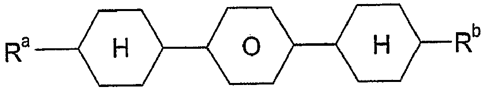 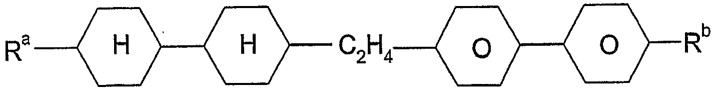 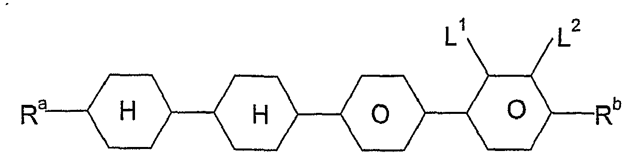 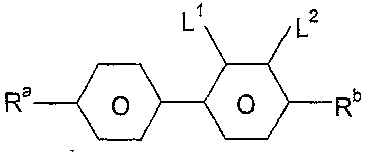 mit R^{a}, R^{b} unabhängig voneinander -CₚH₂ₚ₊₁ oder -OCₚH₂ₚ₊₁ und p = 1, 2, 3, 4, 5, 6, 7 oder 8 sowie L¹, L² unabhängig voneinander -H oder -F, 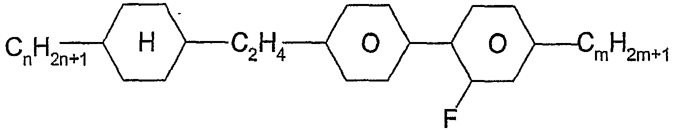 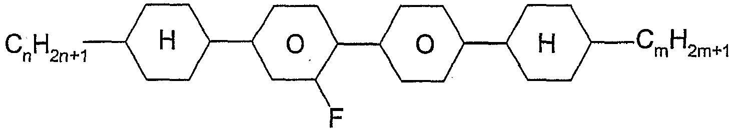 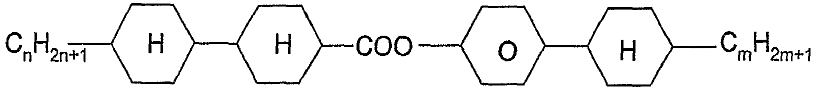 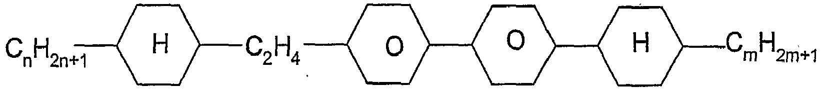 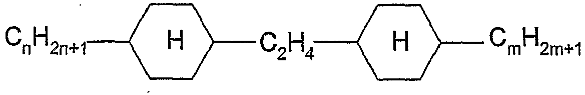 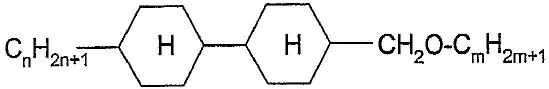 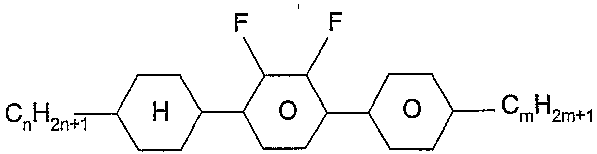 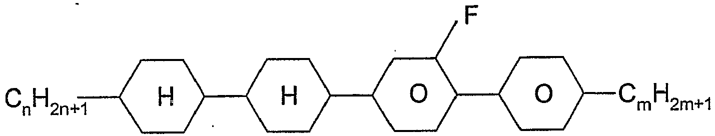 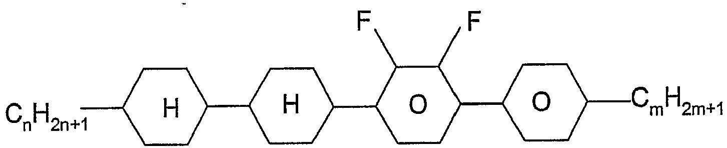 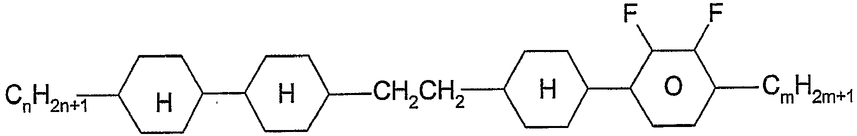 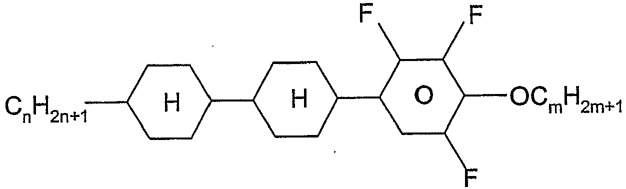 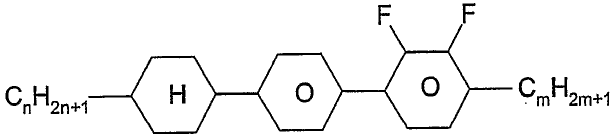 mit m, n unabhängig voneinander 1, 2, 3, 4, 5, 6, 7 oder 8.

Die Herstellung der erfindungsgemäßen Medien erfolgt in an sich üblicher Weise. In der Regel werden die Komponenten ineinander gelöst, vorzugsweise bei erhöhter Temperatur. Durch geeignete Zusätze können die flüssigkristallinen Phasen der vorliegenden Erfindung so modifiziert werden, dass sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die Verbindungen der Formel I eignen sich wegen ihres negativen Δε insbesondere für eine Verwendung in VA-TFT-Displays.

Gegenstand der vorliegenden Erfindung sind daher auch elektrooptische Flüssigkristallanzeigeelemente, enthaltend ein erfindungsgemäßes flüssigkristallines Medium.

Die Erfindung wird im folgenden anhand von Ausführungsbeispielen näher erläutert, ohne dadurch jedoch eingeschränkt zu werden.

Vor- und nachstehend bedeutet Δn die optische Anisotropie (589 nm, 20 °C) und Δε die dielektrische Anisotropie (1 kHz, 20 °C).

Die Δε- und Δn-Werte der erfindungsgemäßen Verbindungen werden durch Extrapolation aus flüssigkristallinen Mischungen erhalten, die zu 10 % aus der jeweiligen erfindungsgemäßen Verbindung und zu 90 % aus der kommerziell erhältlichen Flüssigkristallmischung ZLI-2857 (für Δε) bzw. ZLI-4792 (für Δε) bestehen (Fa. Merck KGaA, Darmstadt). In Fällen begrenzter Löslichkeit wird die Verbindung an einer Mischung gemessen, die nur 5 % der Verbindung enthält, was durch den Zusatz *(5 %)* hinter den betreffenden Werten vermerkt ist.

### Beispiele

Die Ausgangssubstanzen können nach allgemein zugänglichen Literaturvorschriften oder käuflich erhalten werden. Die beschriebenen Reaktionen sind literaturbekannt.

### Beispiel 1

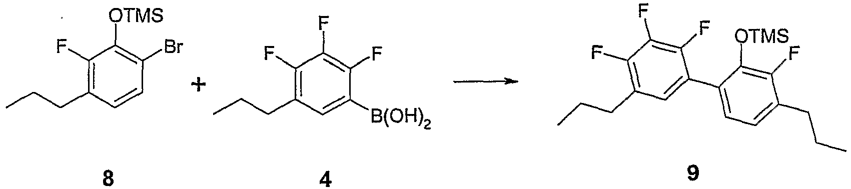

7,0 g (22,9 mmol) des Silylethers 8 werden unter Stickstoff in 20 ml Toluol gelöst, mit 5,0 g (49,3 mmol) Natriumcarbonat, 10 ml Wasser und 175 mg (0,2 mmol) Tetrakis(triphenylphosphin)palladium versetzt. In der Siedehitze wird dem Reaktionsgemisch langsam eine Lösung von 5,0 g (22,9 mmol) der Boronsäure 4 in 10 ml absolutem Ethanol zugesetzt. Nach 5 Stunden Rückfluss und Abkühlen werden die Phasen getrennt. Die wässrige Phase wird mit MTB-Ether extrahiert. Die organische Phase wird über Natriumsulfat getrocknet und eingeengt. Der Rückstand wird an Kieselgel chromatographiert. Man erhält 6,5 g des Silylethers 9 (Ausbeute: 71 %).

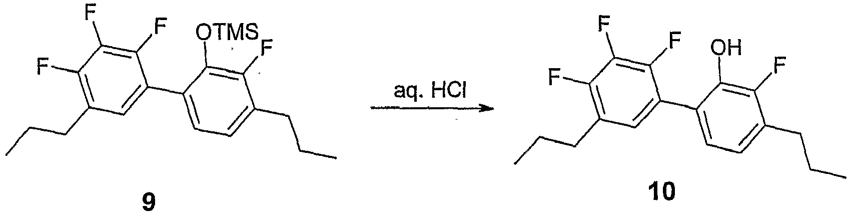

5,0 g (12,6 mmol) des Silylethers 9 werden in 40 ml THF gelöst und mit 10 ml verd. HCl versetzt. Die Lösung wird bis zum vollständigen Umsatz (DC-Kontrolle) bei Raumtemperatur gerührt. Anschließend wird das Lösungsmittel entfernt, und der Rückstand wird in 10 ml verd. HCl-Lösung und 20 ml MTB-Ether aufgenommen. Die wässrige Phase wird dreimal mit MTB-Ether extrahiert. Die organische Phase wird getrocknet, eingeengt und über Kieselgel gegeben. Man erhält 3,7 g des Phenols 10 (Ausbeute: 90 %).

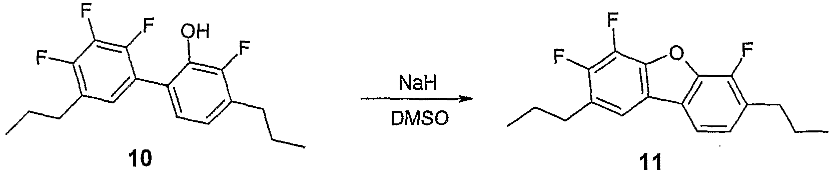

3,0 g (9,2 mmol) des Phenols 10 werden unter Stickstoff in 40 ml DMSO gelöst, mit 736 mg (18,4 mmol) einer 60 %igen Natriumhydrid-Mineralölsuspension versetzt und auf 120°C erwärmt. Nach 5 Stunden bei dieser Temperatur wird der abgekühlte Ansatz in verdünnte HCl-Lösung gegeben. Die wässrige Phase wird mit MTB-Ether extrahiert. Die organische Phase wird über Natriumsulfat getrocknet, eingeengt und an Kieselgel chromatographiert. Man erhält 1,8 g des Dibenzofurans 11 (Ausbeute: 62 %).
Δε: -4,3
Δn: 0,064

Analog Beispiel 1 werden unter Verwendung der entsprechenden Vorstufen die folgenden erfindungsgemäßen Verbindungen erhalten:

### Beispiele 2 bis 64

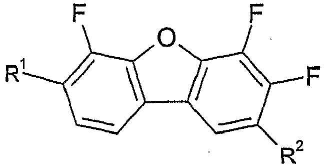

| **Beispiel-Nr.** | **R¹** | **R²** | **Δε** | **Δn** |
|---|---|---|---|---|
| 2 | H | H | | |
| 3 | H | CH₃ | | |
| 4 | H | C₂H₅ | | |
| 5 | H | n-C₃H₇ | | |
| 6 | H | n-C₄H₉ | | |
| 7 | H | n-C₅H₁₁ | | |
| 8 | H | n-C₆H₁₃ | | |
| 9 | H | n-C₇H₁₅ | | |
| 10 | CH₃ | H | | |
| 11 | CH₃ | CH₃ | | |
| 12 | CH₃ | C₂H₅ | | |
| 13 | CH₃ | n-C₃H₇ | | |
| 14 | CH₃ | n-C₄H₉ | | |
| 15 | CH₃ | n-C₅H₁₁ | | |
| 16 | CH₃ | n-C₆H₁₃ | | |
| 17 | CH₃ | n-C₇H₁₅ | | |
| 18 | C₂H₅ | H | | |
| 19 | C₂H₅ | CH₃ | | |
| 20 | C₂H₅ | C₂H₅ | | |
| 21 | C₂H₅ | n-C₃H₇ | | |
| 22 | C₂H₅ | n-C₄H₉ | | |
| 23 | C₂H₅ | n-C₅H₁₁ | | |
| 24 | C₂H₅ | n-C₆H₁₃ | | |
| 25 | C₂H₅ | n-C₇H₁₅ | | |
| 26 | n-C₃H₇ | H | -2,8 | 0,110 |
| 27 | n-C₃H₇ | CH₃ | | |
| 28 | n-C₃H₇ | C₂H₅ | | |
| 29 | n-C₃H₇ | n-C₄H₉ | | |
| 30 | n-C₃H₇ | n-C₅H₁₁ | | |
| 31 | n-C₃H₇ | n-C₆H₁₃ | | |
| 32 | n-C₃H₇ | n-C₇H₁₅ | | |
| 33 | n-C₄H₉ | H | | |
| 34 | n-C₄H₉ | CH₃ | | |
| 35 | n-C₄H₉ | C₂H₅ | | |
| 36 | n-C₄H₉ | n-C₃H₇ | | |
| 37 | n-C₄H₉ | n-C₄H₉ | | |
| 38 | n-C₄H₉ | n-C₅H₁₁ | | |
| 39 | n-C₄H₉ | n-C₆H₁₃ | | |
| 40 | n-C₄H₉ | n-C₇H₁₅ | | |
| 41 | n-C₅H₁₁ | H | | |
| 42 | n-C₅H₁₁ | CH₃ | | |
| 43 | n-C₆H₁₁ | C₂H₅ | | |
| 44 | n-C₅H₁₁ | n-C₃H₇ | | |
| 45 | n-C₅H₁₁ | n-C₄H₉ | | |
| 46 | n-C₅H₁₁ | n-C₅H₁₁ | | |
| 47 | n-C₅H₁₁ | n-C₆H₁₃ | | |
| 48 | n-C₅N₁₁ | n-C₇H₁₅ | | |
| 49 | n-C₆H₁₃ | H | | |
| 50 | n-C₆H₁₃ | CH₃ | | |
| 51 | n-C₆H₁₃ | C₂H₅ | | |
| 52 | n-C₆H₁₃ | n-C₃H₇ | | |
| 53 | n-C₆H₁₃ | n-C₄H₉ | | |
| 54 | n-C₆H₁₃ | n-C₅H₁₁ | | |
| 55 | n-C_{S}H₁₃ | n-C₆H₁₃ | | |
| 56 | n-C₆H₁₃ | n-C₇H₁₅ | | |
| 57 | n-C₇H₁₅ | H | | |
| 58 | n-C₇H₁₅ | CH₃ | | |
| 59 | n-C₇H₁₅ | C₂H₅ | | |
| 60 | n-C₇H₁₅ | n-C₃H₇ | | |
| 61 | n-C₇H₁₅ | n-C₄H₉ | | |
| 62 | n-C₇H₁₅ | n-C₅H₁₁ | | |
| 63 | n-C₇H₁₅ | n-C₆H₁₃ | | |
| 64 | n-C₇H₁₅ | n-C₇H₁₅ | | |

### Beispiele 65 bis 128

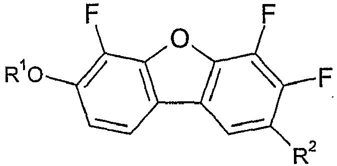

| **Beispiel-Nr.** | **R¹** | **R²** | **Δε** | **Δn** |
|---|---|---|---|---|
| 65 | H | H | | |
| 66 | H | CH₃ | | |
| 67 | H | C₂H₅ | | |
| 68 | H | n-C₃H₇ | | |
| 69 | H | n-C₄H₉ | | |
| 70 | H | n-C₅H₁₁ | | |
| 71 | H | n-C₆H₁₃ | | |
| 72 | H | n-C₇H₁₅ | | |
| 73 | CH₃ | H | | |
| 74 | CH₃ | CH₃ | | |
| 75 | CH₃ | C₂H₅ | | |
| 76 | CH₃ | n-C₃H₇ | | |
| 77 | CH₃ | n-C₄H₉ | | |
| 78 | CH₃ | n-C₅H₁₁ | | |
| 79 | CH₃ | n-C₆H₁₃ | | |
| 80 | CH₃ | n-C₇H₁₅ | | |
| 81 | C₂H₅ | H | -7,8 | 0,149 (5 %) |
| 82 | C₂H₅ | CH₃ | | |
| 83 | C₂H₅ | C₂H₅ | | |
| 84 | C₂H₅ | n-C₃H₇ | -9,1 | 0,107(5%) |
| 85 | C₂H₅ | n-C₄H₉ | | |
| 86 | C₂H₅ | n-C₅H₁₁ | -8,4 | 0,088 |
| 87 | C₂H₅ | n-C₆H₁₃ | | |
| 88 | C₂H₅ | n-C₇H₁₅ | | |
| 89 | n-C₃H₇ | H | | |
| 90 | n-C₃H₇ | CH₃ | | |
| 91 | n-C₃H₇ | C₂H₅ | | |
| 92 | n-C₃H₇ | n-C₃H₇ | | |
| 93 | n-C₃H₇ | n-C₄H₉ | | |
| 94 | n-C₃H₇ | n-C₅H₁₁ | | |
| 95 | n-C₃H₇ | n-C₆H₁₃ | | |
| 96 | n-C₃H₇ | n-C₇H₁₅ | | |
| 97 | n-C₄H₉ | H | | |
| 98 | n-C₄H₉ | CH₃ | | |
| 99 | n-C₄H₉ | C₂H₅ | | |
| 100 | n-C₄H₉ | n-C₃H₇ | | |
| 101 | n-C₄H₉ | n-C₄H₉ | | |
| 102 | n-C₄H₉ | n-C₅H₁₁ | | |
| 103 | n-C₄H₉ | n-C₆H₁₃ | | |
| 104 | n-C₄H₉ | n-C₇H₁₅ | | |
| 105 | n-C₅H₁₁ | H | | |
| 106 | n-C₅H₁₁ | CH₃ | | |
| 107 | n-C₅H₁₁ | C₂H₅ | | |
| 108 | n-C₅H₁₁ | n-C₃H₇ | | |
| 109 | n-C₅H₁₁ | n-C₄H₉ | | |
| 110 | n-C₅H₁₁ | n-C₅H₁₁ | | |
| 111 | n-C₅H₁₁ | n-C₆H₁₃ | | |
| 112 | n-C₅H₁₁ | n-C₇H₁₅ | | |
| 113 | n-C₆H₁₃ | H | | |
| 114 | n-C₆H₁₃ | CH₃ | | |
| 115 | n-C₆H₁₃ | C₂H₅ | | |
| 116 | n-C₆H₁₃ | n-C₃H₇ | | |
| 117 | n-C₆H₁₃ | n-C₄H₉ | | |
| 118 | n-C₆H₁₃ | n-C₅H₁₁ | | |
| 119 | n-C₆H₁₃ | n-C₆H₁₃ | | |
| 120 | n-C₆N₁₃ | n-C₇H₁₅ | | |
| 121 | n-C₇H₁₆ | H | | |
| 122 | n-C₇H₁₅ | CH₃ | | |
| 123 | n-C₇H₁₅ | C₂H₅ | | |
| 124 | n-C₇H₁₅ | n-C₃H₇ | | |
| 125 | n-C₇H₁₅ | n-C₄H₉ | | |
| 126 | n-C₇H₁₅ | n-C₅H₁₁ | | |
| 127 | n-C₇H₁₅ | n-C₆H₁₃ | | |
| 128 | n-C₇H₁₅ | n-C₇H₁₅ | | |

### Beispiele 129 bis 192

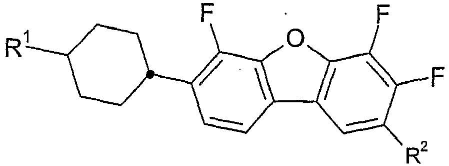

### Beispiele 193 bis 256

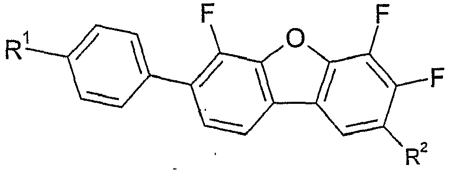

| **Beispiel-Nr.** | | **R¹** | **R²** |
|---|---|---|---|
| 129, | 193 | H | H |
| 130, | 194 | H | CH₃ |
| 131, | 195 | H | C₂H₅ |
| 132, | 196 | H | n-C₃H₇ |
| 133, | 197 | H | n-C₄H₉ |
| 134, | 198 | H | n-C₅H₁₁ |
| 135, | 199 | H | n-C₆H₁₃ |
| 136, | 200 | H | n-C₇H₁₅ |
| 137, | 201 | CH₃ | H |
| 138, | 202 | CH₃ | CH₃ |
| 139, | 203 | CH₃ | C₂H₅ |
| 140, | 204 | CH₃ | n-C₃H₇ |
| 141, | 205 | CH₃ | n-C₄H₉ |
| 142, | 206 | CH₃ | n-C₅H₁₁ |
| 143, | 207 | CH₃ | n-C₆H₁₃ |
| 144, | 208 | CH₃ | n-C₇H₁₅ |
| 145, | 209 | C₂H₅ | H |
| 146, | 210 | C₂H₅ | CH₃ |
| 147, | 211 | C₂H₅ | C₂H₅ |
| 148, | 212 | C₂H₅ | n-C₃H₇ |
| 149, | 213 | C₂H₅ | n-C₄H₉ |
| 150, | 214 | C₂H₅ | n-C₅H₁₁ |
| 151, | 215 | C₂H₅ | n-C₆H₁₃ |
| 152, | 216 | C₂H₅ | n-C₇H₁₅ |
| 153, | 217 | n-C₃H₇ | H |
| 154, | 218 | n-C₃H₇ | CH₃ |
| 155, | 219 | n-C₃H₇ | C₂H₅ |
| 156, | 220 | n-C₃H₇ | n-C₃H₇ |
| 157, | 221 | n-C₃H₇ | n-C₄H₉ |
| 158, | 222 | n-C₃H₇ | n-C₅H₁₁ |
| 159, | 223 | n-C₃H₇ | n-C₆H₁₃ |
| 160, | 224 | n-C₃H₇ | n-C₇H₁₅ |
| 161, | 225 | n-C₄H₉ | H |
| 162, | 226 | n-C₄H₉ | CH₃ |
| 163, | 227 | n-C₄H₉ | C₂H₅ |
| 164, | 228 | n-C₄H₉ | n-C₃H₇ |
| 165, | 229 | n-C₄H₉ | n-C₄H₉ |
| 166, | 230 | n-C₄H₉ | n-C₅H₁₁ |
| 167, | 231 | n-C₄H₉ | n-C₆H₁₃ |
| 168, | 232 | n-C₄H₉ | n-C₇H₁₅ |
| 169, | 233 | n-C₅H₁₁ | H |
| 170, | 234 | n-C₅H₁₁ | CH₃ |
| 171, | 235 | n-C₅H₁₁ | C₂H₅ |
| 172, | 236 | n-C₅H₁₁ | n-C₃H₇ |
| 173, | 237 | n-C₅H₁₁ | n-C₄H₉ |
| 174, | 238 | n-C₅H₁₁ | n-C₅H₁₁ |
| 175, | 239 | n-C₅H₁₁ | n-C₆H₁₃ |
| 176, | 240 | n-C₅H₁₁ | n-C₇H₁₅ |
| 177, | 241 | n-C₆H₁₃ | H |
| 178, | 242 | n-C₆H₁₃ | CH₃ |
| 179, | 243 | n-C₅H₁₃ | C₂H₅ |
| 180, | 244 | n-C₆H₁₃ | n-C₃H₇ |
| 181, | 245 | n-C₆H₁₃ | n-C₄H₉ |
| 182, | 246 | n-C₆H₁₃ | n-C₅H₁₁ |
| 183, | 247 | n-C₆H₁₃ | n-C₆H₁₃ |
| 184, | 248 | n-C₆H₁₃ | n-C₇H₁₅ |
| 185, | 249 | n-C₇H₁₅ | H |
| 186, | 250 | n-C₇H₁₅ | CH₃ |
| 187, | 251 | n-C₇H₁₅ | C₂H₅ |
| 188, | 252 | n-C₇H₁₅ | n-C₃H₇ |
| 189, | 253 | n-C₇H₁₅ | n-C₄H₉ |
| 190, | 254 | n-C₇H₁₅ | n-C₅H₁₁ |
| 191, | 255 | n-C₇H₁₅ | n-C₆H₁₃ |
| 192, | 256 | n-C₇H₁₅ | n-C₇H₁₅ |

### Beispiele 257 bis 320

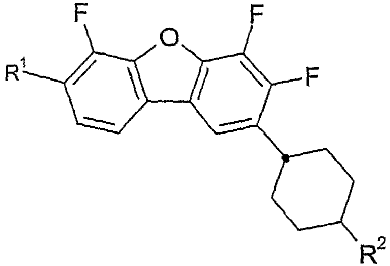

### Beispiele 321 bis 384

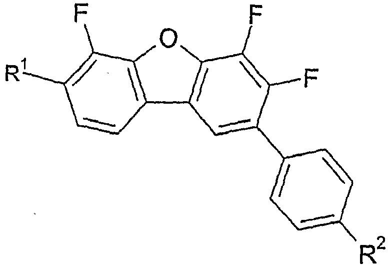

| **Beispiel-Nr.** | | **R¹** | **R²** |
|---|---|---|---|
| 257, | 321 | H | H |
| 258, | 322 | H | CH₃ |
| 259, | 323 | H | C₂H₅ |
| 260, | 324 | H | n-C₃H₇ |
| 261, | 325 | H | n-C₄H₉ |
| 262, | 326 | H | n-C₅H₁₁ |
| 263, | 327 | H | n-C₆H₁₃ |
| 264, | 328 | H | n-C₇H₁₅ |
| 265, | 329 | CH₃ | H |
| 266, | 330 | CH₃ | CH₃ |
| 267, | 331 | CH₃ | C₂H₅ |
| 268, | 332 | CH₃ | n-C₃H₇ |
| 269, | 333 | CH₃ | n-C₄H₉ |
| 270, | 334 | CH₃ | n-C₅H₁₁ |
| 271, | 335 | CH₃ | n-C₆H₁₃ |
| 272, | 336 | CH₃ | n-C₇H₁₅ |
| 273, | 337 | C₂H₅ | H |
| 274, | 338 | C₂H₅ | CH₃ |
| 275, | 339 | C₂H₅ | C₂H₅ |
| 276, | 340 | C₂H₅ | n-C₃H₇ |
| 277, | 341 | C₂H₅ | n-C₄H₉ |
| 278, | 342 | C₂H₅ | n-C₅H₁₁ |
| 279, | 343 | C₂H₅ | n-C₆H₁₃ |
| 280, | 344 | C₂H₅ | n-C₇H₁₅ |
| 281, | 345 | n-C₃H₇ | H |
| 282, | 346 | n-C₃H₇ | CH₃ |
| 283, | 347 | n-C₃H₇ | C₂H₅ |
| 284, | 348 | n-C₃H₇ | n-C₃H₇ |
| 285, | 349 | n-C₃H₇ | n-C₄H₉ |
| 286, | 350 | n-C₃H₇ | n-C₅H₁₁ |
| 287, | 351 | n-C₃H₇ | n-C₆H₁₃ |
| 288, | 352 | n-C₃H₇ | n-C₇H₁₅ |
| 289, | 353 | n-C₄H₉ | H |
| 290, | 354 | n-C₄H₉ | CH₃ |
| 291, | 355 | n-C₄H₉ | C₂H₅ |
| 292, | 356 | n-C₄H₉ | n-C₃H₇ |
| 293, | 357 | n-C₄H₉ | n-C₄H₉ |
| 294, | 358 | n-C₄H₉ | n-C₅H₁₁ |
| 295, | 359 | n-C₄H₉ | n-C₆H₁₃ |
| 296, | 360 | n-C₄H₉ | n-C₇H₁₅ |
| 297, | 361 | n-C₅H₁₁ | H |
| 298, | 362 | n-C₅H₁₁ | CH₃ |
| 299, | 363 | n-C₅H₁₁ | C₂H₅ |
| 300, | 364 | n-C₅H₁₁ | n-C₃H₇ |
| 301, | 365 | n-C₅H₁₁ | n-C₄H₉ |
| 302, | 366 | n-C₅H₁₁ | n-C₅H₁₁ |
| 303, | 367 | n-C₅H₁₁ | n-C₆H₁₃ |
| 304, | 368 | n-C₅H₁₁ | n-C₇H₁₅ |
| 305, | 369 | n-C₆H₁₃ | H |
| 306, | 370 | n-C₆H₁₃ | CH₃ |
| 307, | 371 | n-C₆H₁₃ | C₂H₅ |
| 308, | 372 | n-C₆H₁₃ | n-C₃H₇ |
| 309, | 373 | n-C₆H₁₃ | n-C₄H₉ |
| 310, | 374 | n-C₆H₁₃ | n-C₅H₁₁ |
| 311, | 375 | n-C₆H₁₃ | n-C₆H₁₃ |
| 312, | 376 | n-C₆H₁₃ | n-C₇H₁₅ |
| 313, | 377 | n-C₇H₁₅ | H |
| 314, | 378 | n-C₇H₁₅ | CH₃ |
| 315, | 379 | n-C₇H₁₅ | C₂H₅ |
| 316, | 380 | n-C₇H₁₅ | n-C₃H₇ |
| 317, | 381 | n-C₇H₁₅ | n-C₄H₉ |
| 318, | 382 | n-C₇H₁₅ | n-C₅H₁₁ |
| 319, | 383 | n-C₇H₁₅ | n-C₆H₁₃ |
| 320, | 384 | n-C₇H₁₅ | n-C₇H₁₅ |

### Beispiele 385 bis 448

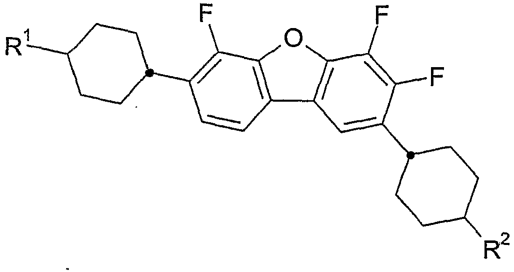

### Beispiele 449 bis 512

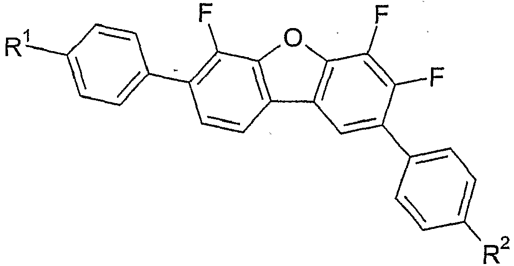

| **Beispiel-Nr.** | | **R¹** | **R²** |
|---|---|---|---|
| 385, | 449 | H | H |
| 386, | 450 | H | CH₃ |
| 387, | 451 | H | C₂H₅ |
| 388, | 452 | H | n-C₃H₇ |
| 389, | 453 | H | n-C₄H₉ |
| 390, | 454 | H | n-C₅H₁₁ |
| 391, | 455 | H | n-C₆H₁₃ |
| 392, | 456 | H | n-C₇H₁₅ |
| 393, | 457 | CH₃ | H |
| 394, | 458 | CH₃ | CH₃ |
| 395, | 459 | CH₃ | C₂H₅ |
| 396, | 460 | CH₃ | n-C₃H₇ |
| 397, | 461 | CH₃ | n-C₄H₉ |
| 398, | 462 | CH₃ | n-C₅H₁₁ |
| 399, | 463 | CH₃ | n-C₆H₁₃ |
| 400, | 464 | CH₃ | n-C₇H₁₅ |
| 401, | 465 | C₂H₅ | H |
| 402, | 466 | C₂H₅ | CH₃ |
| 403, | 467 | C₂H₅ | C₂H₅ |
| 404, | 468 | C₂H₅ | n-C₃H₇ |
| 405, | 469 | C₂H₅ | n-C₄H₉ |
| 406, | 470 | C₂H₅ | n-C₅H₁₁ |
| 407, | 471 | C₂H₅ | n-C₆H₁₃ |
| 408, | 472 | C₂H₅ | n-C₇H₁₅ |
| 409, | 473 | n-C₃H₇ | H |
| 410, | 474 | n-C₃H₇ | CH₃ |
| 411, | 475 | n-C₃H₇ | C₂H₅ |
| 412, | 476 | n-C₃H₇ | n-C₃H₇ |
| 413, | 477 | n-C₃H₇ | n-C₄H₉ |
| 414, | 478 | n-C₃H₇ | n-C₅H₁₁ |
| 415, | 479 | n-C₃H₇ | n-C₆H₁₃ |
| 416, | 480 | n-C₃H₇ | n-C₇H₁₅ |
| 417, | 481 | n-C₄H₉ | H |
| 418, | 482 | n-C₄H₉ | CH₃ |
| 419, | 483 | n-C₄N₉ | C₂H₅ |
| 420, | 484 | n-C₄H₉ | n-C₃H₇ |
| 421, | 485 | n-C₄H₉ | n-C₄N₉ |
| 422, | 486 | n-C₄H₉ | n-C₅H₁₁ |
| 423, | 487 | n-C₄N₉ | n-C₆H₁₃ |
| 424, | 488 | n-C₄N₉ | n-C₇H₁₅ |
| 425, | 489 | n-C₅H₁₁ | H |
| 426, | 490 | n-C₅H₁₁ | CH₃ |
| 427, | 491 | n-C₅H₁₁ | C₂H₅ |
| 428, | 492 | n-C₅H₁₁ | n-C₃H₇ |
| 429, | 493 | n-C₅H₁₁ | n-C₄N₉ |
| 430, | 494 | n-C₅H₁₁ | n-C₅H₁₁ |
| 431, | 495 | n-C₅H₁₁ | n-C₆H₁₃ |
| 432, | 496 | n-C₅H₁₁ | n-C₇H₁₅ |
| 433, | 497 | n-C₆H₁₃ | H |
| 434, | 498 | n-C₆H₁₃ | CH₃ |
| 435, | 499 | n-C₆H₁₃ | C₂H₅ |
| 436, | 500 | n-C₆H₁₃ | n-C₃H₇ |
| 437, | 501 | n-C₆H₁₃ | n-C₄H₉ |
| 438, | 502 | n-C₆H₁₃ | n-C₅H₁₁ |
| 439, | 503 | n-C₆H₁₃ | n-C₆H₁₃ |
| 440, | 504 | n-C₆H₁₃ | n-C₇H₁₅ |
| 441, | 505 | n-C₇H₁₅ | H |
| 442, | 506 | n-C₇H₁₅ | CH₃ |
| 443, | 507 | n-C₇H₁₅ | C₂H₅ |
| 444, | 508 | n-C₇H₁₅ | n-C₃H₇ |
| 445, | 509 | n-C₇H₁₅ | n-C₄H₉ |
| 446, | 510 | n-C₇H₁₅ | n-C₅H₁₁ |
| 447, | 511 | n-C₇H₁₅ | n-C₆H₁₃ |
| 448, | 512 | n-C₇H₁₅ | n-C₇H₁₅ |

### Beispiele 513 bis 576

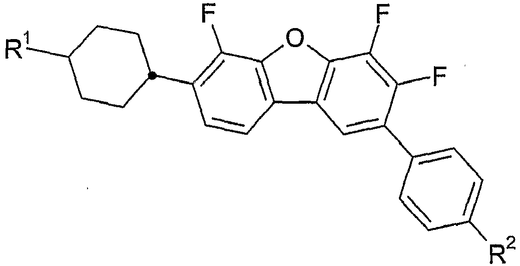

### Beispiele 577 bis 640

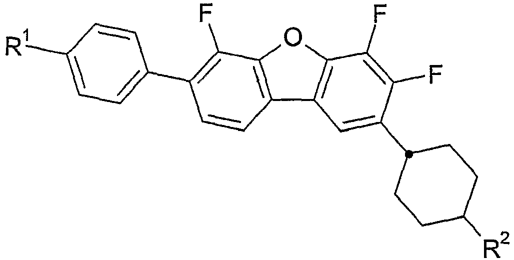

| **Beispiel-Nr.** | | **R¹** | **R²** |
|---|---|---|---|
| 513, | 577 | H | H |
| 514, | 578 | H | CH₃ |
| 515, | 579 | H | C₂H₅ |
| 516, | 580 | H | n-C₃H₇ |
| 517, | 581 | H | n-C₄H₉ |
| 518, | 582 | H | n-C₅H₁₁ |
| 519, | 583 | H | n-C₆H₁₃ |
| 520, | 584 | H | n-C₇H₁₅ |
| 521, | 585 | CH₃ | H |
| 522, | 586 | CH₃ | CH₃ |
| 523, | 587 | CH₃ | C₂H₅ |
| 524, | 588 | CH₃ | n-C₃H₇ |
| 525, | 589 | CH₃ | n-C₄H₉ |
| 526, | 590 | CH₃ | n-C₅H₁₁ |
| 527, | 591 | CH₃ | n-C₆H₁₃ |
| 528, | 592 | CH₃ | n-C₇H₁₅ |
| 529, | 593 | C₂H₅ | H |
| 530, | 594 | C₂H₅ | CH₃ |
| 531, | 595 | C₂H₅ | C₂H₅ |
| 532, | 596 | C₂H₅ | n-C₃H₇ |
| 533, | 597 | C₂H₅ | n-C₄H₉ |
| 534, | 598 | C₂H₅ | n-C₅H₁₁ |
| 535, | 599 | C₂H₅ | n-C₆H₁₃ |
| 536, | 600 | C₂H₅ | n-C₇H₁₅ |
| 537, | 601 | n-C₃H₇ | H |
| 538, | 602 | n-C₃H₇ | CH₃ |
| 539, | 603 | n-C₃H₇ | C₂H₅ |
| 540, | 604 | n-C₃H₇ | n-C₃H₇ |
| 541, | 605 | n-C₃H₇ | n-C₄H₉ |
| 542, | 606 | n-C₃H₇ | n-C₅H₁₁ |
| 543, | 607 | n-C₃H₇ | n-C₆H₁₃ |
| 544, | 608 | n-C₃H₇ | n-C₇H₁₅ |
| 545, | 609 | n-C₄H₉ | H |
| 546, | 610 | n-C₄H₉ | CH₃ |
| 547, | 611 | n-C₄H₉ | C₂H₅ |
| 548, | 612 | n-C₄H₉ | n-C₃H₇ |
| 549, | 613 | n-C₄H₉ | n-C₄H₉ |
| 550, | 614 | n-C₄H₉ | n-C₅H₁₁ |
| 551, | 615 | n-C₄H₉ | n-C₆H₁₃ |
| 552, | 616 | n-C₄H₉ | n-C₇H₁₅ |
| 553, | 617 | n-C₅H₁₁ | H |
| 554, | 618 | n-C₅H₁₁ | CH₃ |
| 555, | 619 | n-C₅H₁₁ | C₂H₅ |
| 556, | 620 | n-C₅H₁₁ | n-C₃H₇ |
| 557, | 621 | n-C₅H₁₁ | n-C₄H₉ |
| 558, | 622 | n-C₅H₁₁ | n-C₅H₁₁ |
| 559, | 623 | n-C₅H₁₁ | n-C₆H₁₃ |
| 560, | 624 | n-C₅H₁₁ | n-C₇H₁₅ |
| 561, | 625 | n-C₆H₁₃ | H |
| 562, | 626 | n-C₆H₁₃ | CH₃ |
| 563, | 627 | n-C₆H₁₃ | C₂H₅ |
| 564, | 628 | n-C₆H₁₃ | n-C₃H₇ |
| 565, | 629 | n-C₆H₁₃ | n-C₄H₉ |
| 566, | 630 | n-C₆H₁₃ | n-C₅H₁₁ |
| 567, | 631 | n-C₆H₁₃ | n-C₆H₁₃ |
| 568, | 632 | n-C₆H₁₃ | n-C₇H₁₅ |
| 569, | 633 | n-C₇H₁₅ | H |
| 570, | 634 | n-C₇H₁₅ | CH₃ |
| 571, | 635 | n-C₇H₁₅ | C₂H₅ |
| 572, | 636 | n-C₇H₁₅ | n-C₃H₇ |
| 573, | 637 | n-C₇H₁₅ | n-C₄H₉ |
| 574, | 638 | n-C₇H₁₅ | n-C₅H₁₁ |
| 575, | 639 | n-C₇H₁₅ | n-C₆H₁₃ |
| 576, | 640 | n-C₇H₁₅ | n-C₇H₁₅ |

### Beispiel 641

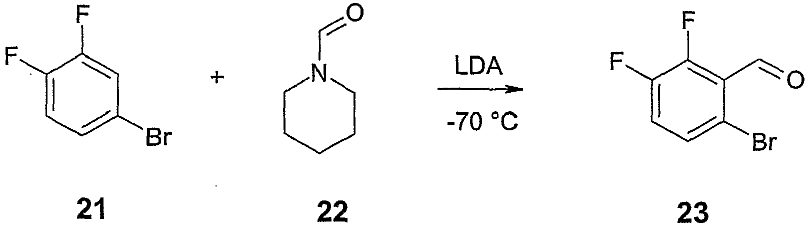

Unter Stickstoff und bei -70 °C wird eine Lösung von 77,2 g (400 mmol) des Aromaten **21** in 500 ml THF mit 220 ml (440 mmol) einer 2 M Lösung von Lithiumdiisopropylamid in Cyclohexan/Ethylbenzol/THF versetzt. Nach 1,5 Stunden bei der tiefen Temperatur werden bei -50°C bis -40 °C 55,5 ml (500 mmol) N-Formylpiperidin **22** dem Ansatz hinzugefügt. Nach 3 Stunden bei -40 °C wird der Ansatz bei 0 °C hydrolysiert und mit Salzsäure angesäuert. Die wässrige Phase wird mit MTB-Ether extrahiert, die organische Phase wird mit ges. Natriumchlorid-Lösung gewaschen, über Natriumsulfat getrocknet, eingeengt und über Kieselgel gegeben (MTB-Ether/n-Heptan 1:20). Man erhält 72,1 g (81 %) des Aldehyds **23.**

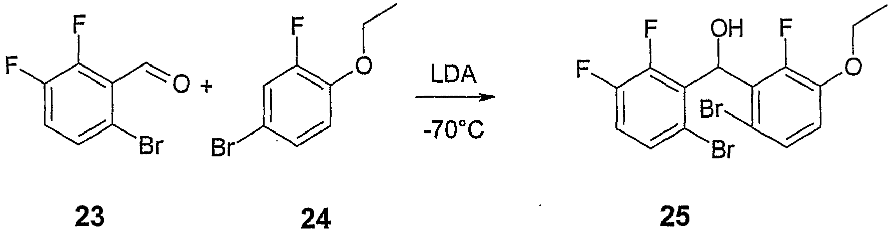

Unter Stickstoff und bei -70 °C wird eine Lösung von 24,0 g (110 mmol) des Aromaten **24** in 150 ml THF mit 16,1 ml (120 mmol) einer 2 M Lösung von Lithiumdiisopropylamid in Cyclohexan/Ethylbenzol/THF versetzt. Nach 1,5 Stunden bei der tiefen Temperatur werden 22,1 g (100 mmol) des Aldehyds **23** gelöst in 50 ml THF dem Ansatz hinzugefügt. Nach 3 Stunden bei -70 °C wird der Ansatz auf Raumtemperatur erwärmt, hydrolysiert und mit Salzsäure angesäuert. Die wässrige Phase wird mit MTB-Ether extrahiert, die organische Phase wird mit ges. Natriumchlorid-Lösung gewaschen, über Natriumsulfat getrocknet, eingeengt und über Kieselgel gegeben (MTB-Ether/n-Heptan 1:10). Man erhält 40,2 g (83 %) des Diphenylmethanols **25.**

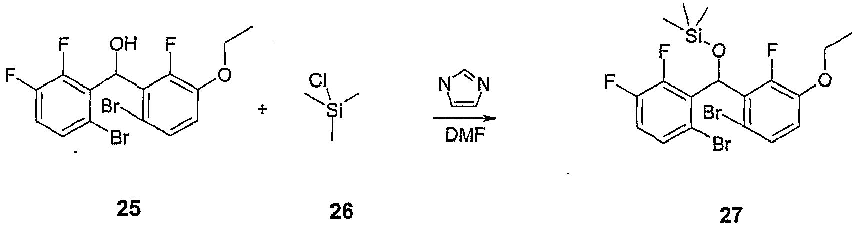

Unter Stickstoff werden 14,7 g (213 mmol) Imidazol und 35,0 g (79,5 mmol) des Diphenylmethanols **25** in 100 ml DMF gelöst, mit 14,4 ml Trimethylsilylchlorid **26** versetzt und bei Raumtemperatur über Nacht gerührt. Der Ansatz wird mit ges. Natriumchlorid-Lösung versetzt und mit MTB-Ether extrahiert. Die organische Phase wird über Natriumsulfat getrocknet, eingeengt und über Kieselgel gegeben (MTB-Ether/n-Heptan 1:20). Man erhält 37,9 g (93 %) des Silylethers **27.**

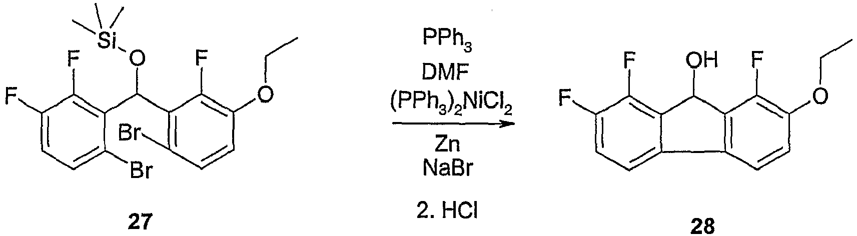

Unter Stickstoff werden 12,4 g (189 mmol) Zink, 19,6 g (30 mmol) Bis-triphenylphosphin-Nickel(II)chlorid, 18,5 g (180 mmol) Natriumbromid und 47,6 g (180 mmol) Triphenylphosphin mit 200 ml DMF versetzt und auf 80 °C erwärmt. Nach 30 Minuten werden 30,7 g (60 mmol) des Silylethers **27** gelöst in 50 ml DMF tropfenweise in den Ansatz gegeben. Nach 3 Tagen wird der abgekühlte Ansatz mit Wasser versetzt und MTB-Ether extrahiert. Die organische Phase wird mit ges. Natriumchlorid-Lösung gewaschen über Natriumsulfat getrocknet und eingeengt. Der Rückstand wird in THF aufgenommen und mit konz. Salzsäure versetzt. Nach vollständigem Umsatz (DC) wird der Ansatz mit ges. Natriumchlorid-Lösung versetzt und mit MTB-Ether extrahiert. Die organische Phase wird mit ges. Natrium-chlorid-Lösung gewaschen, über Natriumsulfat getrocknet und eingeengt. Nach Chromatographie (MTB-Ether/n-Heptan 1:1) an Kieselgel erhält man 2,5 g (15 %) des Fluorenols **28.**

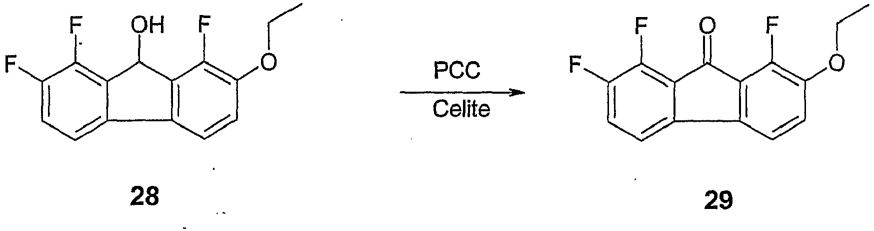

Unter Stickstoff werden 15 g Pyridiniumchlorochromat (PCC) in eine Suspension von 40 g Celite in 150 ml Dichlormethan gegeben. Anschließend wird eine Lösung von 10,0 g (35,6 mmol) des Fluorenols **28** in 25 ml Dichlormethan in die Suspension gegeben. Der Ansatz wird über Nacht bei Raumtemperatur gerührt. Das Celite wird abgetrennt und mit Dichlormethan gewaschen. Die organische Phase wird eingeengt und über Kieselgel gegeben (MTB-Ether/n-Heptan 1:3). Man erhält 9,0 g (91 %) des Fluorenons **29.**

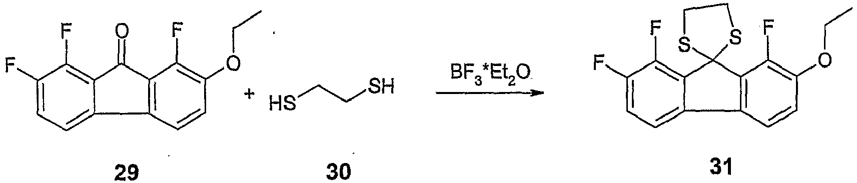

Unter Stickstoff wird bei -10 °C eine Lösung von 8,5 g (30,6 mmol) des Fluorenons **29** und 3,8 ml (45 mmol) Ethandithiol **30** in 50 ml Dichlormethan mit 16,1 ml Bortrifluorid-Diethylether-Komplex versetzt. Der Ansatz taut über Nacht auf und wird vorsichtig auf ges. Natriumhydrogencarbonat-Lösung gegeben. Der pH Wert wird auf 8 eingestellt. Die wässrige Phase wird mit Dichlormethan extrahiert, die organische Phase wird mit ges. Natriumchlorid-Lösung gewaschen, über Natriumsulfat getrocknet und eingeengt. Der Rückstand wird über Kieselgel gegeben (MTB-Ether/n-Heptan 1:15). Man erhält 9,6 g (89 %) des Thioketals **31.**

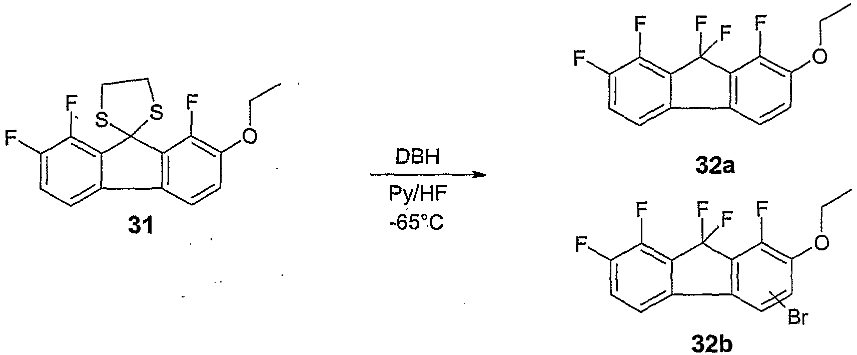

Unter Stickstoff und bei einer Temperatur von unter -65°C wird eine Suspension von 28,6 g (100 mmol) 1,3-Dibrom-5,5-dimethylhydantoin in 150 ml Dichlormethan und 29,2 ml einer 65 %igen Lösung von HF in Pyridin mit einer Lösung von 9,0 g (25,4 mmol) des Thioketals **31** in 50 ml Dichlormethan versetzt. Nach 5 Stunden wird die Kühlung entfernt und der Ansatz rührt über Nacht. Anschließend wird der Ansatz auf 11 eisgekühlte 1 N Natronlauge, die mit 35 ml 19 %iger Natriumhydrogensulfit-Lösung versetzt worden ist, gegeben. Der pH wird auf 8 eingestellt. Die wässrige Phase wird mit Dichlormethan extrahiert, die organische Phase wird mit ges. Natriumchlorid-Lösung gewaschen, über Natriumsulfat getrocknet, eingeengt und über Kieselgel gegeben (MTB-Ether/n-Heptan 1:30). Man erhält 8 g eines Gemisches der Fluorene **32a** und **32b.**

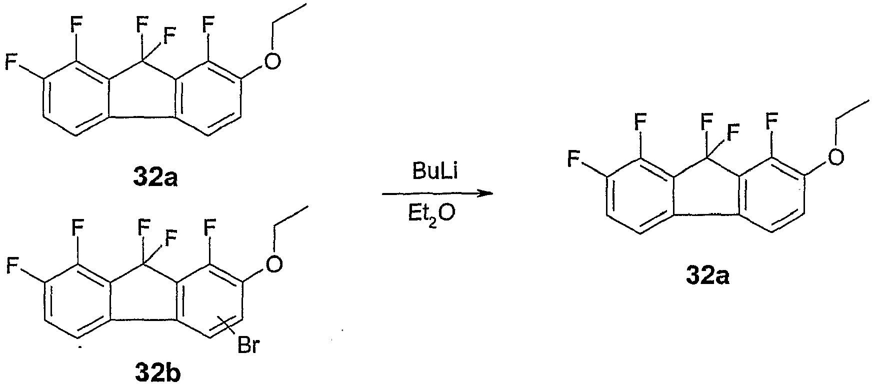

8 g des Fluorengemisches werden unter Stickstoff in 50 ml Diethylether gelöst und bei -70°C mit 15,7 ml (25,0 mmol) einer 15 %igen Lösung von Butyllithium in n-Hexan versetzt. Nach 1 Stunde werden 10 ml einer 1:1-THF/Wasser-Mischung der Lösung zugesetzt und der Ansatz auf Raumtemperatur erwärmt. Der Ansatz wird mit ges. Natriumchlorid-Lösung gewaschen, über Natriumsulfat getrocknet und eingeengt. Der erhaltene Rückstand wird über Kieselgel gegeben (MTB-Ether/n-Heptan 1:30). Man erhält 5,3 g (70 % bez. auf die vorherige Stufe) des Fluorens **32a.**

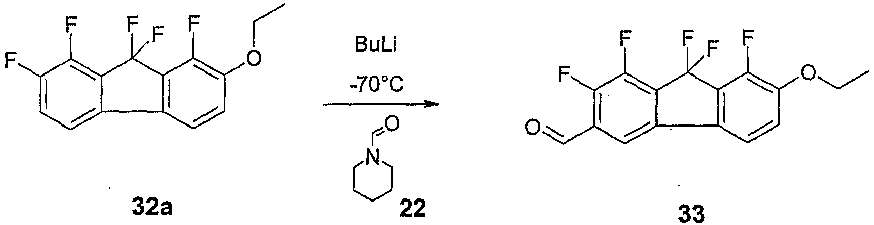

Unter Stickstoff und bei -70 °C wird eine Lösung von 5,0 g (16,7 mmol) des Fluorens **32a** in 75 ml THF mit 10,7 ml (17,0 mmol) einer 15 %igen Lösung von Butyllithium in n-Hexan versetzt. Nach 1,5 Stunden bei der tiefen Temperatur werden bei -50 °C bis -40 °C 2,2 ml (20 mmol) N-Formylpiperidin **22** dem Ansatz hinzugefügt. Nach 3 Stunden bei -40 °C wird der Ansatz bei 0 °C hydrolysiert und mit Salzsäure angesäuert. Die wässrige Phase wird mit MTB-Ether extrahiert, die organische Phase wird mit ges. Natriumchlorid-Lösung gewaschen, über Natriumsulfat getrocknet, eingeengt und über Kieselgel gegeben (MTB-Ether/n-Heptan 1:15). Man erhält 4,3 g (79 %) des Aldehyds **33.**

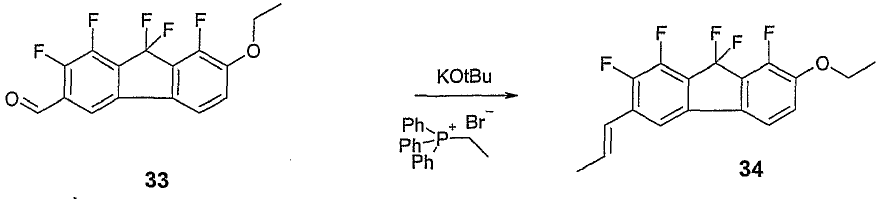

Unter Stickstoff wird eine Suspension von 6,0 g (16,2 mmol) Ethyl-tristriphenylphosphoniumbromid in 30 ml THF bei 0°C mit einer Lösung von 1,7 g (15 mmol) Kalium-*tert*-Butylat 15 ml THF versetzt. Nach 1 Stunde wird der Aldehyd 33 gelöst in 10 ml THF langsam zugegeben, wobei die Ansatztemperatur 10 °C nicht übersteigt. Der Ansatz wird über Nacht bei Raumtemperatur gerührt und anschließend mit Wasser versetzt. Die wässrige Phase wird mit MTB-Ether extrahiert, die organische Phase wird mit ges. Natriumchlorid-Lösung gewaschen, über Natriumsulfat getrocknet und eingeengt. Der Rückstand wird über Kieselgel gegeben (MTB-Ether/n-Heptan 1:15). Man erhält 3,7 g (79 %) des Olefins **34.**

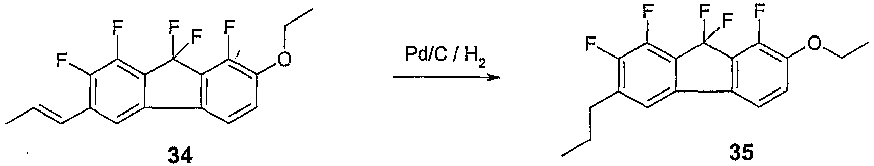

3,5 g (10,3 mmol) des Olefins **34** werden in 40 ml THF gelöst und am Palladiumkatalysator hydriert. Die Hydrierlösung wird eingeengt. Der erhaltene Rückstand wird über Kieselgel gegeben. Man erhält 3,2 g (92 %) des Pentafluorfluorens **35.**

Analog Beispiel 641 werden unter Verwendung der entsprechenden Vorstufen die folgenden erfindungsgemäßen Verbindungen erhalten:

### Beispiele 642 bis 704

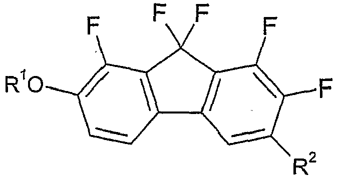

### Beispiele 705 bis 768

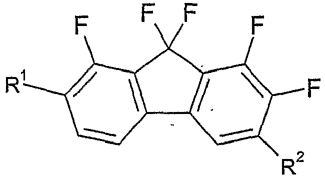

| **Beispiel-Nr.** | | **R¹** | **R²** |
|---|---|---|---|
| 641, | 705 | C₂H₅ | n-C₃H₇ |
| 642, | 706 | H | H |
| 643, | 707 | H | CH₃ |
| 644, | 708 | H | C₂H₅ |
| 645, | 709 | H | n-C₃H₇ |
| 646, | 710 | H | n-C₄H₉ |
| 647, | 711 | H | n-C₅H₁₁ |
| 648, | 712 | H | n-C₆H₁₃ |
| 649, | 713 | H | n-C₇H₁₅ |
| 650, | 714 | CH₃ | H |
| 651, | 715 | CH₃ | CH₃ |
| 652, | 716 | CH₃ | C₂H₅ |
| 653, | 717 | CH₃ | n-C₃H₇ |
| 654, | 718 | CH₃ | n-C₄H₉ |
| 655, | 719 | CH₃ | n-C₅H₁₁ |
| 656, | 720 | CH₃ | n-C₆H₁₃ |
| 657, | 721 | CH₃ | n-C₇H₁₅ |
| 658, | 722 | C₂H₅ | H |
| 659, | 723 | C₂H₅ | CH₃ |
| 660, | 724 | C₂H₅ | C₂H₅ |
| 661, | 725 | C₂H₅ | n-C₄H₉ |
| 662, | 726 | C₂H₅ | n-C₅H₁₁ |
| 663, | 727 | C₂H₅ | n-C₆H₁₃ |
| 664, | 728 | C₂H₅ | n-C₇H₁₅ |
| 665, | 729 | n-C₃H₇ | H |
| 666, | 730 | n-C₃H₇ | CH₃ |
| 667, | 731 | n-C₃H₇ | C₂H₅ |
| 668, | 732 | n-C₃H₇ | n-C₃H₇ |
| 669, | 733 | n-C₃H₇ | n-C₄H₉ |
| 670, | 734 | n-C₃H₇ | n-C₅H₁₁ |
| 671, | 735 | n-C₃H₇ | n-C₆H₁₃ |
| 672, | 736 | n-C₃H₇ | n-C₇H₁₅ |
| 673, | 737 | n-C₄H₉ | H |
| 674, | 738 | n-C₄H₉ | CH₃ |
| 675, | 739 | n-C₄H₉ | C₂H₅ |
| 676, | 740 | n-C₄H₉ | n-C₃H₇ |
| 677, | 741 | n-C₄H₉ | n-C₄H₉ |
| 678, | 742 | n-C₄H₉ | n-C₅H₁₁ |
| 679, | 743 | n-C₄H₉ | n-C₆H₁₃ |
| 680, | 744 | n-C₄H₉ | n-C₇H₁₅ |
| 681, | 745 | n-C₅H₁₁ | H |
| 682, | 746 | n-C₅H₁₁ | CH₃ |
| 683, | 747 | n-C₅H₁₁ | C₂H₅ |
| 684, | 748 | n-C₅H₁₁ | n-C₃H₇ |
| 685, | 749 | n-C₅H₁₁ | n-C₄H₉ |
| 686, | 750 | n-C₅H₁₁ | n-C₅H₁₁ |
| 687, | 751 | n-C₅H₁₁ | n-C₆H₁₃ |
| 688, | 752 | n-C₅H₁₁ | n-C₇H₁₅ |
| 689, | 753 | n-C₆H₁₃ | H |
| 690, | 754 | n-C₆H₁₃ | CH₃ |
| 691, | 755 | n-C₆H₁₃ | C₂H₅ |
| 692, | 756 | n-C₆H₁₃ | n-C₃H₇ |
| 693, | 757 | n-C₆H₁₃ | n-C₄H₉ |
| 694, | 758 | n-C₆H₁₃ | n-C₅H₁₁ |
| 695, | 759 | n-C₆H₁₃ | n-C₆H₁₃ |
| 696, | 760 | n-C₆H₁₃ | n-C₇H₁₅ |
| 697, | 761 | n-C₇H₁₅ | H |
| 698, | 762 | n-C₇H₁₅ | CH₃ |
| 699, | 763 | n-C₇H₁₅ | C₂H₅ |
| 700, | 764 | n-C₇H₁₅ | n-C₃H₇ |
| 701, | 765 | n-C₇H₁₅ | n-C₄H₉ |
| 702, | 766 | n-C₇H₁₅ | n-C₅H₁₁ |
| 703, | 767 | n-C₇H₁₅ | n-C₆H₁₃ |
| 704, | 768 | n-C₇H₁₅ | n-C₇H₁₅ |

## Patentansprüche

1. Verbindung der allgemeinen Formel I: 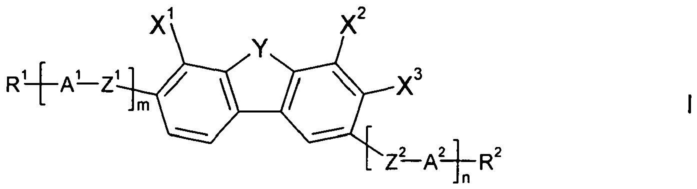 worin:
m und n jeweils unabhängig voneinander 0, 1, 2 oder 3 sind;
Y O, S, S(O), SO₂, CH₂, CF₂, CCl₂, CHF, CHCl, CFCl, C(CF₃)₂, CHCF₃, C(CN)₂ oder CHCN bedeutet;
X¹, X² und X³ F bedeuten;
A¹ und A² jeweils unabhängig voneinander 1,4-Phenylen, worin =CH- ein- oder zweimal durch =N- ersetzt sein kann und das unsubstituiert oder ein- bis viermal unabhängig voneinander mit -CN, -F, -Cl, -Br, -I, unsubstituiertem oder mit Fluor und/oder Chlor ein- oder mehrfach substituiertem C₁-C₆-Alkanyl, unsubstituiertem oder mit Fluor und/oder Chlor ein- oder mehrfach substituiertem C₁-C₆-Alkoxy substituiert sein kann, 1,4-Cyclohexylen, 1,4-Cyclohexenylen oder 1,4-Cyclohexadienylen, worin -CH₂- ein- oder zweimal unabhängig voneinander durch -O- oder -S- so ersetzt sein kann, dass Heteroatome nicht direkt verknüpft sind, und die unsubstituiert oder ein- oder mehrfach durch -F, -Cl, -Br und/oder -I substituiert sein können, bedeutet;
Z¹ und Z² jeweils unabhängig voneinander eine Einfachbindung, eine Doppelbindung, -CF₂O-, -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- oder -C≡C-bedeuten;
R¹ und R² Wasserstoff, einen unsubstituierten, einen einfach mit -CN oder -CF₃ oder einen einfach oder mehrfach mit F, Cl, Br und/oder I substituierten Alkanyl-, Alkoxy-, Alkenyl- oder Alkinylrest mit 1 bis 15 bzw. 2 bis 15 C-Atomen, wobei in diesen Resten auch eine oder mehrere CH₂-Gruppen jeweils unabhängig voneinander durch -O-, -S-, -SO₂-, -CO-, -COO-, -OCO- oder -OCO-O- so ersetzt sein können, dass Heteroatome nicht direkt verknüpft sind, -F, -Cl, -Br, -I, -CN, -SCN, -NCS oder -SF₅ bedeuten;
wobei
A¹, A²; Z¹, Z²; R¹, R² jeweils die gleiche oder unterschiedliche Bedeutungen haben können, wenn m beziehungsweise n größer als 1 sind.

2. Verbindung gemäß Anspruch 1, **dadurch gekennzeichnet, dass** (m + n) ≤ 3 ist.

3. Verbindung gemäß Anspruch 1 oder 2, **dadurch gekennzeichnet, dass** Y = O oder CF₂ ist.

4. Verbindung gemäß wenigstens einem der vorangehenden Ansprüche, **dadurch gekennzeichnet, dass**
Z¹ und Z² unabhängig voneinander eine Einfachbindung, -CF₂O-, -OCF₂-, -CF₂CF₂-, -CH=CH-, -CF=CH-, -CH=CF- oder -CF=CF- sind.

5. Verbindung gemäß wenigstens einem der vorangehenden Ansprüche, **dadurch gekennzeichnet, dass**
A¹ und A² unabhängig voneinander 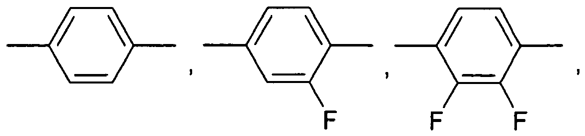 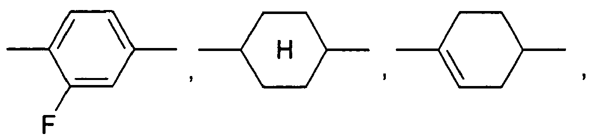 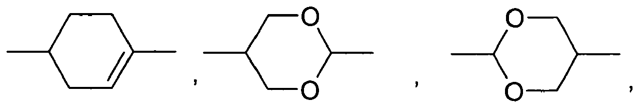 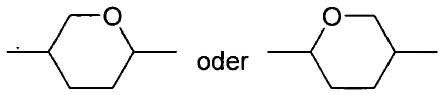 sind.

6. Verbindung gemäß wenigstens einem der vorangehenden
Ansprüche, **dadurch gekennzeichnet, dass**
R¹ und R² jeweils unabhängig voneinander ein Alkanylrest, Alkoxyrest oder Alkenylrest mit 1 bis 7 bzw. 2 bis 7 Kohlenstoffatomen sind, wobei jeder dieser Reste unsubstituiert oder mit Halogen einfach oder mehrfach substituiert ist, oder Wasserstoff bedeutet.

7. Verbindung gemäß wenigstens einem der vorangehenden
Ansprüche, **dadurch gekennzeichnet, dass**
m und n beide null sind; und
R¹ und R² jeweils unabhängig voneinander ein unverzweigter Alkanylrest, Alkoxyrest oder Alkenylrest mit 1 bis 7 bzw. 2 bis 7 Kohlenstoffatomen sind.

8. Verbindung gemäß wenigstens einem der Ansprüche 1 bis 6, **dadurch gekennzeichnet, dass**
m = 1 ist und n = 0 ist;
A¹ 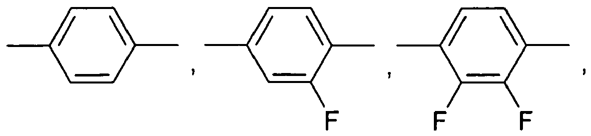 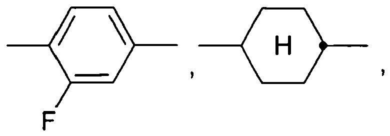 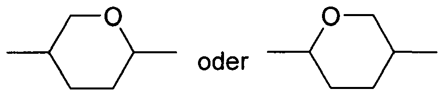 ist;
Z¹ eine Einfachbindung, -CF₂O-, -OCF₂-, -CF₂CF₂-, -CH=CH-, -CF=CH-, -CH=CF- oder -CF=CF- ist;
R¹ ein Alkanylrest, Alkoxyrest oder Alkenylrest mit 1 bis 7 bzw. 2 bis 7 Kohlenstoffatomen ist; und
R² Wasserstoff oder ein Alkanylrest, Alkoxyrest oder Alkenylrest mit 1 bis 7 bzw. 2 bis 7 Kohlenstoffatomen ist.

9. Verbindung gemäß wenigstens einem der Ansprüche 1 bis 6, **dadurch gekennzeichnet, dass**
m und n = 1 sind;
A¹ und A² beide 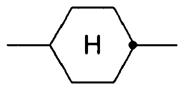 sind;
Z¹ und Z² eine Einfachbindung sind;
R¹ ein Alkanylrest, Alkoxyrest oder Alkenylrest mit 1 bis 7 bzw. 2 bis 7 Kohlenstoffatomen ist; und
R² Wasserstoff oder ein Alkanylrest, Alkoxyrest oder Alkenylrest mit 1 bis 7 bzw. 2 bis 7 Kohlenstoffatomen ist.

10. Verwendung einer Verbindung gemäß wenigstens einem der vorangehenden Ansprüche in flüssigkristallinen Medien.

11. Flüssigkristallines Medium mit mindestens zwei flüssigkristallinen Verbindungen, **dadurch gekennzeichnet, dass** es mindestens eine Verbindung gemäß wenigstens einem der Ansprüche 1 bis 9 enthält.

12. Elektrooptisches Anzeigeelement, enthaltend ein flüssigkristallines Medium gemäß Anspruch 11.

## Claims

1. Compound of the general formula I: 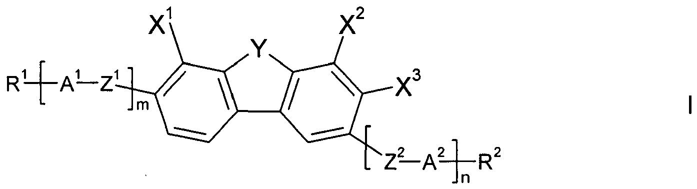 in which:
m and n are each, independently of one another, 0, 1, 2 or 3;
Y denotes O, S, S(O), SO₂, CH₂, CF₂, CCl₂, CHF, CHCl, CFCl, C(CF₃)₂, CHCF₃, C(CN)₂ or CHCN;
X¹, X² and X³ denote F;
A¹ and A² each, independently of one another, denote 1,4-phenylene, in which =CH- may be replaced once or twice by =N- and which may be unsubstituted or mono- to tetrasubstituted, independently of one another, by -CN, -F, -Cl, -Br, -I, C₁-C₆-alkanyl which is unsubstituted or mono- or polysubstituted by fluorine and/or chlorine, C₁-C₆-alkoxy which is unsubstituted or mono- or polysubstituted by fluorine and/or chlorine, 1,4-cyclohexylene, 1,4-cyclohexenylene or 1,4-cyclohexadienylene, in which -CH₂- may be replaced once or twice, independently of one another, by -O- or -S- in such a way that heteroatoms are not linked directly, and which may be unsubstituted or mono- or polysubstituted by -F, -Cl, -Br and/or -I;
Z¹ and Z² each, independently of one another, denote a single bond, a double bond, -CF₂O-, -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- or -C≡C-;
R¹ and R² denote hydrogen, an alkanyl, alkoxy, alkenyl or alkynyl radical having 1 to 15 or 2 to 15 C atoms respectively which is unsubstituted, monosubstituted by -CN or -CF₃ or monosubstituted or polysubstituted by F, Cl, Br and/or I, where, in addition, one or more CH₂ groups in these radicals may each be replaced, independently of one another, by -O-, -S-, -SO₂-, -CO-, -COO-, -OCO- or -OCO-O- in such a way that heteroatoms are not linked directly, -F, -Cl, -Br, -I, -CN, -SCN, -NCS or -SF₅;
where
A¹, A²; Z¹, Z²; R¹, R² may each have identical or different meanings if m or n is greater than 1.

2. Compound according to Claim 1, **characterised in that** (m+n) is ≤ 3.

3. Compound according to Claim 1 or 2, **characterised in that** Y = O or CF₂.

4. Compound according to at least one of the preceding claims, **characterised in that**
Z¹ and Z², independently of one another, are a single bond, -CF₂O-, -OCF₂-, -CF₂CF₂-, -CH=CH-, -CF=CH-, -CH=CF- or -CF=CF-.

5. Compound according to at least one of the preceding claims, **characterised in that**
A¹ and A², independently of one another, are

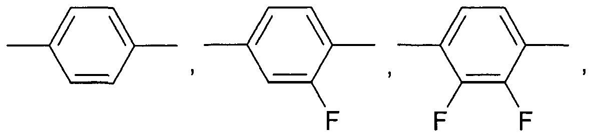

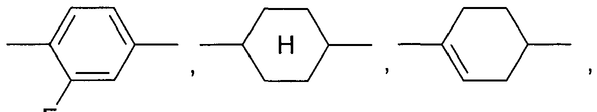

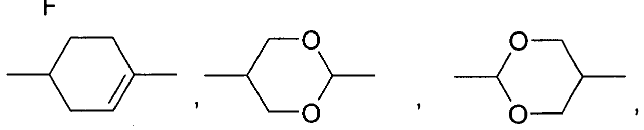

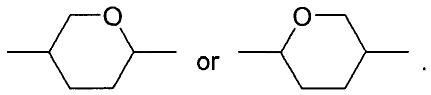

6. Compound according to at least one of the preceding claims, **characterised in that**
R¹ and R² are each, independently of one another, an alkanyl radical, alkoxy radical or alkenyl radical having 1 to 7 or 2 to 7 carbon atoms respectively, where each of these radicals is unsubstituted or monosubstituted or polysubstituted by halogen, or denotes hydrogen.

7. Compound according to at least one of the preceding claims, **characterised in that**
m and n are both zero; and
R¹ and R² are each, independently of one another, an unbranched alkanyl radical, alkoxy radical or alkenyl radical having 1 to 7 or 2 to 7 carbon atoms respectively.

8. Compound according to at least one of Claims 1 to 6, **characterised in that**
m = 1 and n = 0;
A¹ is 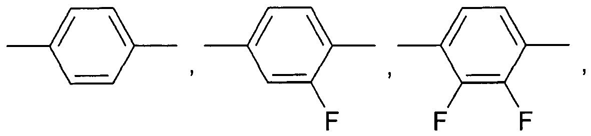 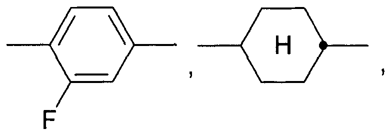 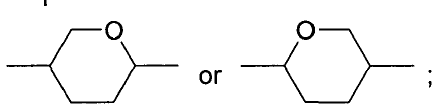
Z¹ is a single bond, -CF₂O-, -OCF₂-, -CF₂CF₂-, -CH=CH-, -CF=CH-, -CH=CF- or -CF=CF-;
R¹ is an alkanyl radical, alkoxy radical or alkenyl radical having 1 to 7 or 2 to 7 carbon atoms respectively; and
R² is hydrogen or an alkanyl radical, alkoxy radical or alkenyl radical having 1 to 7 or 2 to 7 carbon atoms respectively.

9. Compound according to at least one of Claims 1 to 6, **characterised**
**in that**
m and n = 1;
A¹ and A² are both 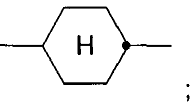
Z¹ and Z² are a single bond;
R¹ is an alkanyl radical, alkoxy radical or alkenyl radical having 1 to 7 or 2 to 7 carbon atoms respectively; and
R² is hydrogen or an alkanyl radical, alkoxy radical or alkenyl radical having 1 to 7 or 2 to 7 carbon atoms respectively.

10. Use of a compound according to at least one of the preceding claims in liquid-crystalline media.

11. Liquid-crystalline medium comprising at least two liquid-crystalline compounds, **characterised in that** it comprises at least one compound according to at least one of Claims 1 to 9.

12. Electro-optical display element containing a liquid-crystalline medium according to Claim 11.

## Revendications

1. Composé de la formule générale I: 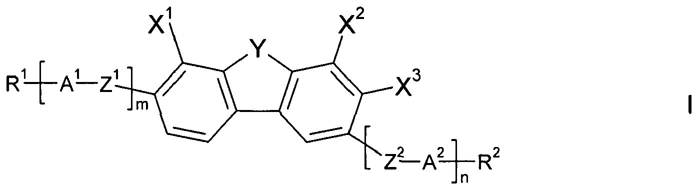 dans laquelle:
m et n sont chacun, indépendamment l'un de l'autre, 0, 1, 2 ou 3;
Y représente O, S, S(O), SO₂, CH₂, CF₂, CCl₂, CHF, CHCl, CFCl, C(CF₃)₂, CHCF₃, C(CN)₂ ou CHCN;
X¹, X² et X³ représentent F;
A¹ et A² chacun, indépendamment l'un de l'autre, représentent 1,4-phénylène, où =CH- peut être remplacé une fois ou deux fois par =N- et lequel peut être non substitué ou mono- à tétrasubstitué, indépendamment l'un de l'autre, par -CN, -F, -Cl, -Br, -I, C₁-C₆-alkanyle qui est non substitué ou mono- ou polysubstitué par fluor et/ou chlore, C₁-C₆-alcoxy qui est non substitué ou mono- ou polysubstitué par fluor et/ou chlore, 1,4-cyclohexylène, 1,4-cyclohexénylène ou 1,4-cyclohexadiénylène, où -CH₂- peut être remplacé une fois ou deux fois, indépendamment l'un de l'autre, par -O- ou -S- de telle sorte que des hétéroatomes ne soient pas liés directement, et qui peut être non substitué ou mono- ou polysubstitué par -F, -Cl, -Br et/ou -I;
Z¹ et Z² chacun, indépendamment l'un de l'autre, représentent une liaison simple, une liaison double, -CF₂O-, -OCF₂-, -CH₂CH₂-, -CF₂CF₂-, -CF₂CH₂-, -CH₂CF₂-, -CHF-CHF-, -C(O)O-, -OC(O)-, -CH₂O-, -OCH₂-, -CF=CH-, -CH=CF-, -CF=CF-, -CH=CH- ou -C≡C-;
R¹ et R² représentent hydrogène, un radical alkanyle, alcoxy, alkényle ou alkynyle ayant respectivement 1 à 15 ou 2 à 15 atomes de C lequel est non substitué, monosubstitué par -CN ou -CF₃ ou monosubstitué ou polysubstitué par F, Cl, Br et/ou I, où, en addition, un ou plusieurs groupes CH₂ dans ces radicaux peuvent chacun être remplacés, indépendamment l'un de l'autre, par -O-, -S-, -SO₂-, -CO-, -COO-, -OCO- ou -OCO-O- de telle sorte que des hétéroatomes ne soient pas liés directement, -F, -Cl, -Br, -I, -CN, -SCN, -NCS ou -SF₅;
où
A¹, A²; Z¹, Z²; R¹, R² peuvent chacun avoir des cycles identiques ou différents si m ou n est supérieur à 1.

2. Composé selon la revendication 1, **caractérisé en ce que** (m + n) est ≤ 3.

3. Composé selon la revendication 1 ou 2, **caractérisé en ce que** Y = O ou CF₂.

4. Composé selon au moins l'une des revendications précédentes, **caractérisé en ce que**
Z¹ et Z², indépendamment l'un de l'autre, sont une liaison simple, -CF₂O-, -OCF₂-, -CF₂CF₂-, -CH=CH-, -CF=CH-, -CH=CF- ou -CF=CF-.

5. Composé selon au moins l'une des revendications précédentes, **caractérisé en ce que**
A¹ et A², indépendamment l'un de l'autre, sont

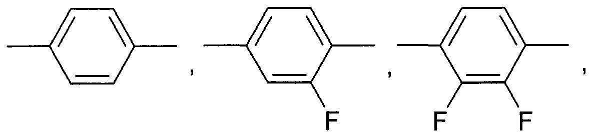

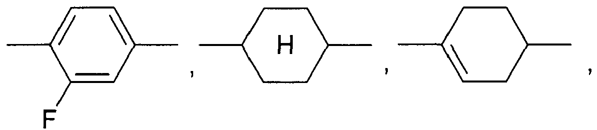

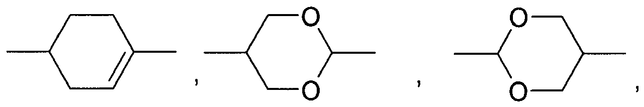

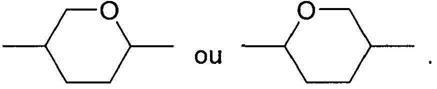

6. Composé selon au moins l'une des revendications précédentes, **caractérisé en ce que**
R¹ et R² sont chacun, indépendamment l'un de l'autre, un radical alkanyle, un radical alcoxy ou un radical alkényle ayant respectivement 1 à 7 ou 2 à 7 atomes de carbone, où chacun de6 ces radicaux est non substitué ou monosubstitué ou polysubstitué par halogène, ou représente hydrogène.

7. Composé selon au moins l'une des revendications précédentes, **caractérisé en ce que**
m et n sont les deux zéro; et
R¹ et R² sont chacun, indépendamment l'un de l'autre, un radical alkanyle non ramifié, un radical alcoxy ou un radical alkényle ayant respectivement 1 à 7 ou 2 à 7 atomes de carbone.

8. Composé selon au moins l'une des revendications 1 à 6, **caractérisé en ce que**
m = 1 et n = 0;
A¹ est 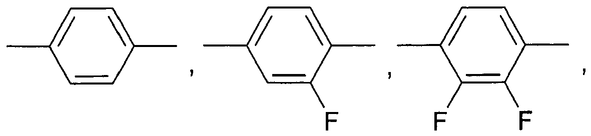 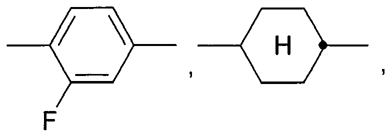 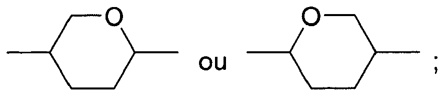 Z¹ est une liaison simple, -CF₂O-, -OCF₂-, -CF₂CF₂-, -CH=CH-, -CF=CH-, -CH=CF- ou -CF=CF-;
R¹ est un radical alkanyle, un radical alcoxy ou un radical alkényle ayant respectivement 1 à 7 ou 2 à 7 atomes de carbone; et
R² est hydrogène ou un radical alkanyle, un radical alcoxy ou un radical alkényle ayant respectivement 1 à 7 ou 2 à 7 atomes de carbone.

9. Composé selon au moins l'une des revendications 1 à 6, **caractérisé**
**en ce que**
m et n = 1;
A¹ et A² sont tous deux 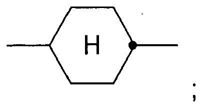
Z¹ et Z² sont une liaison simple;
R¹ est un radical alkanyle, un radical alcoxy ou un radical alkényle ayant respectivement 1 à 7 ou 2 à 7 atomes de carbone; et
R² est hydrogène ou un radical alkanyle, un radical alcoxy ou un radical alkényle ayant respectivement 1 à 7 ou 2 à 7 atomes de carbone.

10. Utilisation d'un composé selon au moins l'une des revendications précédentes dans des milieux cristallins liquides.

11. Milieu cristallin liquide comprenant au moins deux composés cristallins liquides, **caractérisé en ce qu'**il comprend au moins un composé selon au moins l'une des revendications 1 à 9.

12. Elément d'affichage électro-optique contenant un milieu cristallin liquide selon la revendication 11.
